# EUROPEAN PATENT APPLICATION

(11) **EP 3 273 218 A1**
(43) Date of publication of application: **24.01.2018**
(21) Application number: 16764642.1
(22) Date of filing: 23.02.2016
(51) Int. Cl.: G01N 1/30, G01N 1/28, G01N 33/48, G02B 21/33, G02B 21/34

(54) **METHOD FOR OBSERVING BIOLOGICAL MATERIAL AND CLEARING METHOD**

(30) Priority: 18.03.2015 JP 2015055325
(71) Applicant: Riken, Wako-shi, Saitama 351-0198 (JP)
(72) Inventor: IMAI, Takeshi, Wako-shi Saitama 351-0198 (JP); KE, Meng-Tsen, Wako-shi Saitama 351-0198 (JP)
(74) Representative: Graf von Stosch, Andreas
(86) International application number: PCT/JP2016/055229
(87) International publication number: WO 2016/147812

(57) **Abstract**

In an aspect of the present invention, in order to (i) clear the biological material by reducing light scattering while preserving fine morphology of a biological material and preserving fluorescence of a fluorescent substance, and (ii) reduce a spherical aberration in observation under a microscope, the biological material is cleared by being immersed in a solution containing a detergent and a non-ionic organoiodine compound, and then the cleared biological material is observed under a microscope in a state of being immersed in a solution that contains a non-ionic organoiodine compound and that has a refractive index higher than 1.48.

## Description

### Technical Field

The present invention relates to a method for observing a biological material and a method for clearing a biological material.

### Background Art

In recent years, techniques for enabling deep fluorescent observation by clearing biological tissues have been developed one after another. However, it is not easy to observe a deep part with high-resolution. High-resolution fluorescence imaging such as in a super-resolution microscope is extremely easily affected by scattering due to a sample and by a spherical aberration, and it has been particularly difficult to observe a deep part. Under the circumstances, in deep fluorescent observation of biological tissues, further development has been demanded in a clearing method for reducing light scattering and in a mounting agent for reducing a spherical aberration after clearing.

As methods for clearing tissues, for example, Scale method (Non-Patent Literature 1) and CUBIC method (Non-Patent Literature 2) in each of which urea is used are known, and also CLARITY method (Non-Patent Literature 3) and the like are known in which tissues are crosslinked with acrylamide gel and then lipid is removed. Moreover, a reagent FocusClear™ is commercially available (Patent Literature 1). Further, SeeDB method (Non-Patent Literature 4) in which fructose is used is known.

Meanwhile, mounting agents containing glycerin have been widely used but refractive indices of such mounting agents are merely approximately 1.46. In a variant of the CLARITY method, a reagent RIMS (containing iohexol, a phosphate buffer, and a detergent Tween 20) is known which is used to adjust a refractive index after lipid is removed by crosslinking the tissues with hydrogel monomers (Non-Patent Literature 5). However, in a case where fluorescence imaging is performed with use of an oil-immersion objective lens (in which a refractive index of immersion oil is 1.518) and a glass coverslip having a refractive index of 1.518 for achieving high-resolution, it is preferable to mount a tissue sample in a liquid having a high refractive index equivalent to those of the immersion oil and the glass coverslip. As a mounting agent having a high refractive index, a mounting agent containing 2,2'-thiodiethanol (TDE) (with a refractive index of 1.52) is known (Non-Patent Literature 6).

### Citation List

### [Patent Literature]

[Patent Literature 1]

US Patent No. 6472216, specification (Date of Patent: October 29, 2002)

### [Non-patent Literature]

[Non-Patent Literature 1]
   Hama, H., Kurokawa, H., Kawano, H., Ando, R., Shimogori, T., Noda, H., Fukami, K., Sakaue-Sawano, A. & Miyawaki, A. (2011) Nat. Neurosci., 14, 1481-1488.
[Non-Patent Literature 2]
   Susaki, E.A., Tainaka, K., Perrin, D., Kishino, F., Tawara, T., Watanabe, T.M., Yokoyama, C., Onoe, H., Eguchi, M., Yamaguchi, S., Abe, T., Kiyonari, H., Shimizu, Y., Miyawaki, A., Yokota, H. & Ueda, H.R. (2014) Cell, 157, 726-739.
[Non-Patent Literature 3] Chung, K., Wallace, J., Kim, S.Y., Kalyanasundaram, S., Andalman, A.S., Davidson, T.J., Mirzabekov, J.J., Zalocusky, K.A., Mattis, J., Denisin, A.K., Pak, S., Bernstein, H., Ramakrishnan, C., Grosenick, L., Gradinaru, V. & Deisseroth, K. (2013) Nature, 497, 332-337.
[Non-Patent Literature 4]
   Ke, M.T., Fujimoto, S. & Imai, T. (2013) Nat. Neurosci., 16, 1154-1161.
[Non-Patent Literature 5]
   Yang, B., Treweek, J.B., Kulkarni, R.P., Deverman, B.E., Chen, C.K., Lubeck, E., Shah, S., Cai, L. & Gradinaru, V. (2014) Cell, 158, 945-958.
[Non-Patent Literature 6]
   Staudt, T., Lang, M.C., Medda, R., Engelhardt, J. & Hell, S.W. (2007) Microsc. Res. Tech., 70, 1-9.

### Summary of Invention

### Technical Problem

However, the clearing methods disclosed in Non-Patent Literatures 1 through 3 are excellent in transparency but may change fine morphology of tissues.

In a case where the clearing agent disclosed in Patent Literature 1 is used, there is a problem that a sample shrinks.

The clearing method disclosed in Non-Patent Literature 4 can achieve imaging while preserving fine morphology but there is still room for improvement in long-term stability of a fluorescent protein and, in regard to high-resolution imaging, there is a possibility that a sufficient effect cannot be obtained due to restriction of solubility of fructose.

In Non-Patent Literature 5, RIMS which is used in mounting of a sample has a refractive index of 1.38 to 1.48, which is not equivalent to the refractive indices of the immersion oil and the glass coverslip. Therefore, it is impossible to obtain high resolution and brightness in a deep part of a sample.

The mounting agent disclosed in Non-Patent Literature 6 quenches a fluorescent protein and the like, and thus has restriction in fluorescence imaging.

The present invention is accomplished in view of the problems, and its object is to provide a method for clearing a biological material by reducing light scattering of the biological material while preserving fine morphology of the biological material and preserving fluorescence of a fluorescent substance, a method for reducing a spherical aberration in observation under a microscope, and the like.

### Solution to Problem

A method in accordance with an aspect of the present invention for clearing a biological material includes a clearing step A of clearing the biological material by immersing the biological material in a solution that contains a detergent and a non-ionic organoiodine compound.

A method in accordance with an aspect of the present invention for observing a biological material includes an observing step of observing the biological material under a microscope in a state in which the biological material is immersed in a solution that contains a non-ionic organoiodine compound and that has a refractive index higher than 1.48.

A clearing agent in accordance with an aspect of the present invention is a clearing agent for clearing a biological material and is a solution containing a detergent and a non-ionic organoiodine compound.

A mounting agent in accordance with an aspect of the present invention is a mounting agent for use in observing a biological material under a microscope, the mounting agent being a solution that contains a non-ionic organoiodine compound and that has a refractive index higher than 1.48.

A kit in accordance with an aspect of the present invention for observing a biological material is a kit for use in clearing a biological material and observing the biological material under a microscope and includes: a first solution that contains a detergent and a non-ionic organoiodine compound; and a second solution that contains a non-ionic organoiodine compound and that has a refractive index higher than 1.48.

### Advantageous Effects of Invention

The present invention can provide a method for clearing a biological material by reducing light scattering of the biological material while preserving fine morphology of the biological material and preserving fluorescence of a fluorescent substance, a method for reducing a spherical aberration in observation under a microscope, and the like.

### Brief Description of Drawings

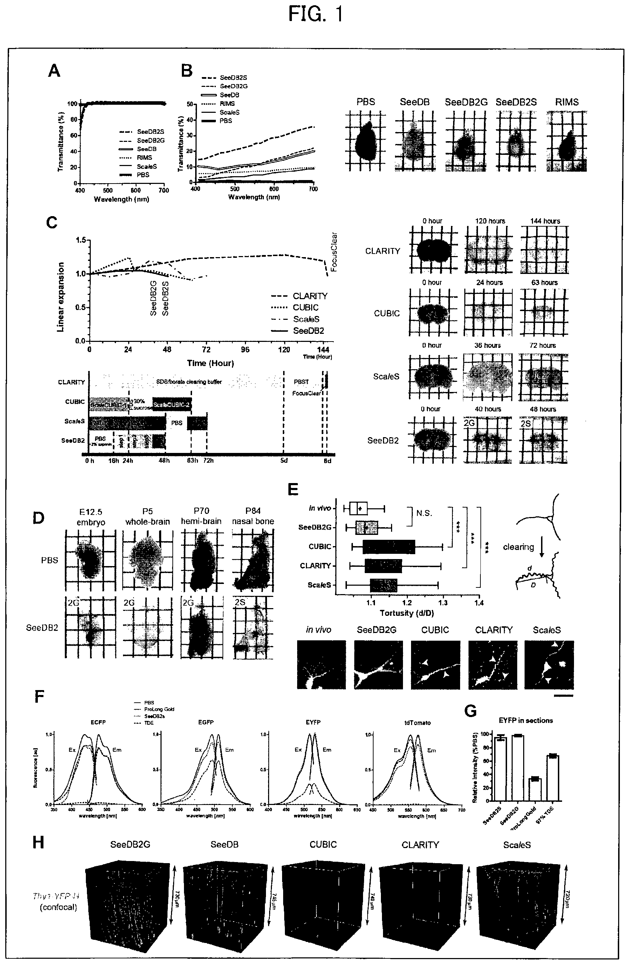

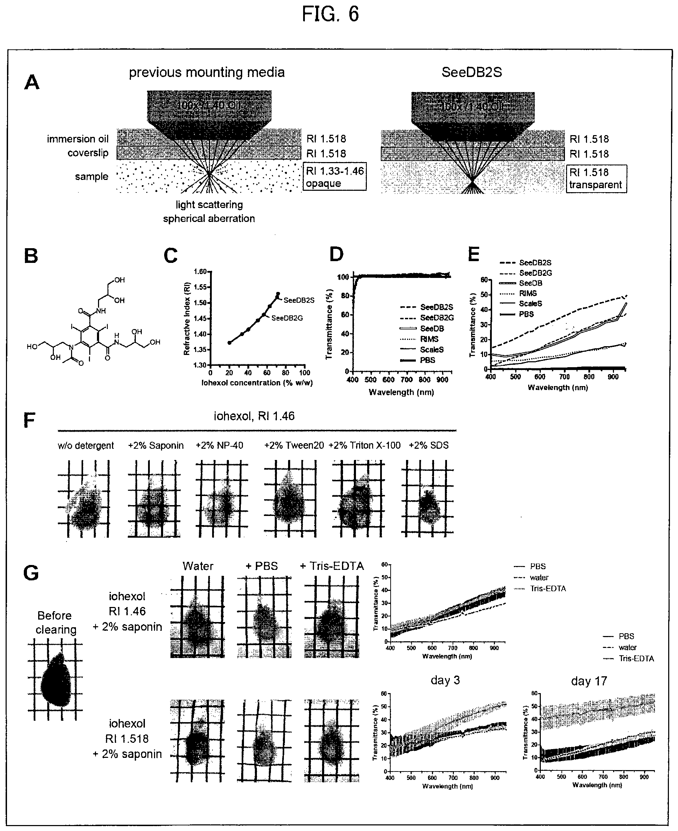

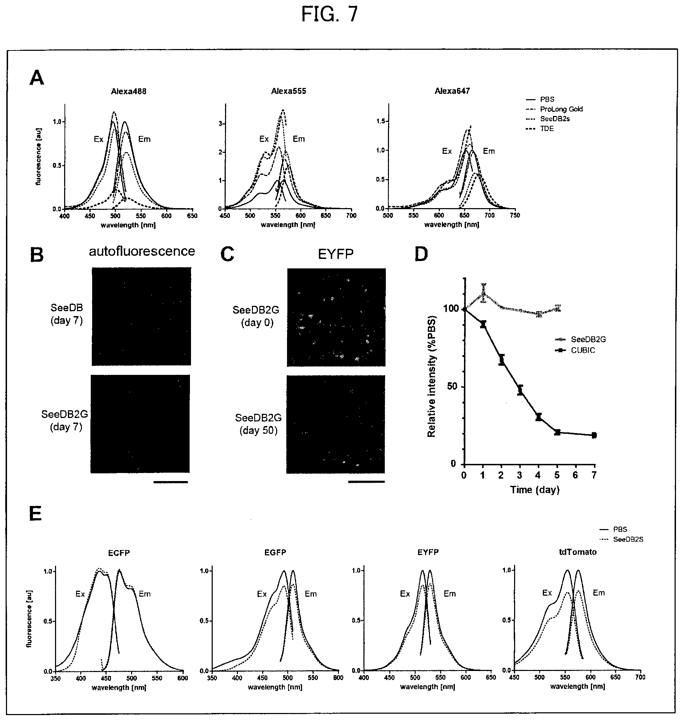

Fig. 1 is a view illustrating that fine morphology and fluorophores are preserved in SeeDB2. (A) Transmission curves of various clearing media in the visual wavelength range. (B) Transmission curves of cerebral cortices of the mouse brains (age, postnatal day (P) 21-24, n = 3-6 each) cleared with various clearing protocols. Representative transmission images are shown on the right. RIMS caused shrinkage of samples without improving transparency. Full-scan data including IR range is shown in D and E of Fig. 6. (C) Schedules of clearing and sample size changes during optical clearing by CLARITY, CUBIC, ScaleS, and SeeDB2. Adult mouse brain slices (P49-56, 1.5 mm thick, n = 3 each) were used, as shown on the right. CLARITY, CUBIC, and ScaleS accompany transient swelling of tissues. In contrast, SeeDB2 was not accompanied by dramatic sample size changes during clearing process. (D) Various samples before and after clearing with SeeDB2. A mouse embryo (E12.5), a whole brain (P5), a hemi-brain (P70), and a nasal bone (P84), before and after optical clearing with SeeDB2 are shown. (E) Deformation of fine neuronal structures. Tortuosities of mitral cell lateral dendritic spine segments were quantified and shown by box plots (n = 43, 81, 68, 58, and 80 from 3-5 animals). Distorted dendrites are indicated by arrowheads. Bars indicate 5th and 95th percentiles. ***P< 0.001 (Kruskal-Wallis Test). N.S., non-significant. Grids in transmission images are 2.6 × 3.2 mm. A scale bar represents 50 µm. (F) Fluorescence excitation (Ex, broken lines) and emanation (Em, solid lines) spectra of fluorescent proteins in various clearing media. Fluorescence levels were normalized to the maximum intensity in PBS. TDE quenched fluorescence in all fluorescent proteins. ProLong Gold partially quenched EGFP and EYFP signals. In contrast, all fluorescent proteins were stable in SeeDB2S. n = 3 each. (G) Stability of EYFP in a cross section of a *Thy1-YFP-G* mouse brain. Quenching in CUBIC is evaluated in (D) of Fig. 7. (H) Confocal imaging of *Thy1-YFP-H* mouse (P72-84) cerebral cortex after various optical clearing methods (representative images). Images were taken from the surface of the hemi-brain samples under the same imaging conditions using a 20× air objective lens. SeeDB2G kept autofluorescence signals low while best preserving fluorescence signals.

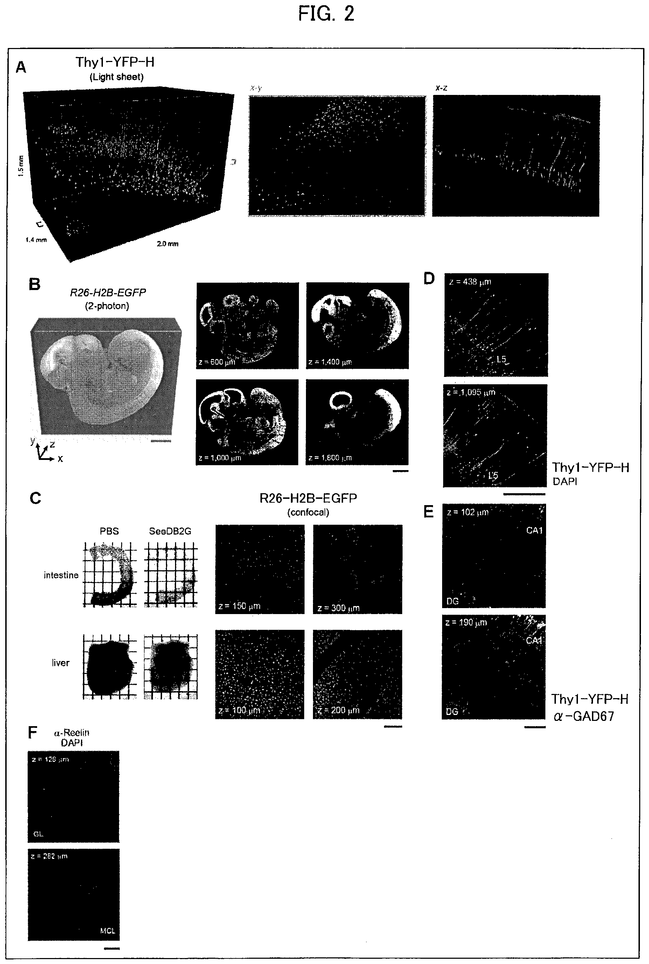

Fig. 2 is a view illustrating clearing and staining of various tissues in SeeDB2. (A) Light-sheet microscopy of adult *Thy1-YFP-H* mouse brain cleared with SeeDB2G (P58). A volume of 1.39 mm × 2.04 mm × 1.49 mm was reconstructed from 2 × 3 blocks, each consisting of 745 images. It took ∼6 min to obtain this image, much faster than confocal or two-photon microscopy (several hours). (B) Two-photon images of *R26-H2B-EGFP* mouse embryo (E9.5). An image of the whole-embryo was reconstructed from 4 × 5 blocks. (C) Optical clearing of intestine and liver from adult *R26-H2B-EGFP* mice. Confocal images are shown on the right. (D) DAPI staining of 1.5 mm-thick brain slices (cerebral cortex) from *Thy1-YFP-H* mouse (P56). (E) Anti-GAD67 immunostaining of the *Thy1-YFP-H* mouse brain slice (hippocampus). (F) Anti-Reelin immunostaining of whole-mount olfactory bulb sample (P56). (D)-(F) are confocal images of SeeDB2G-cleared samples. L5, layer V; DG, dentate gyrus; GL, glomerular layer; MCL, mitral cell. Scale bars represent 1 mm in (B) and 100 µm in (C)-(F).

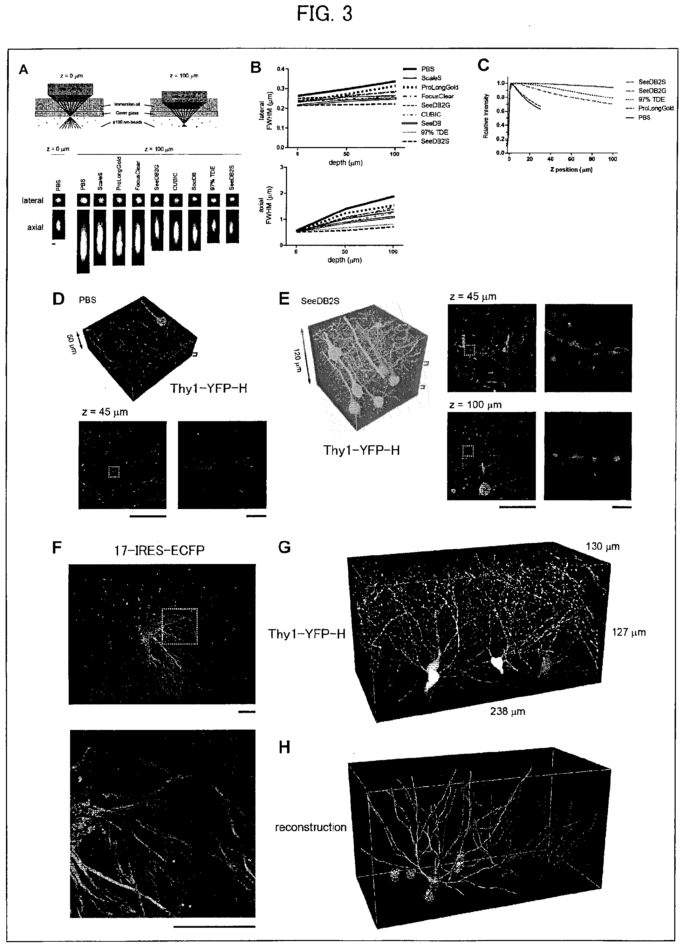

Fig. 3 is a view illustrating resolution and brightness improved in SeeDB2S. (A) Point-spread-function (PSF) analysis of fluorescent microspheres (diameter, 100 nm) in various clearing and mounting media. Fluorescence images of the fluorescent microspheres obtained with confocal microscopy (pinhole size, 1AU) and an oil-immersion objective lens (100×, NA 1.40, WD 0.13 mm) are shown. (B) Total length in full width at half maximum (FWHM) for a lateral direction and a z-axis direction is shown. Best resolution was obtained by SeeDB2S in which the refractive index was identical with those of the glass coverslip and the immersion oil. Note that z-axis resolution was more severely affected by refractive index mismatch. n = 20 each. (C) Confocal axial scans of rhodamine dye solutions from the interface with the glass coverslip. Due to spherical aberrations, photons detected by PMT decrease as the depth increases when the refractive index is mismatched. In contrast, fluorescence intensity was consistent throughout depth when the dye was dissolved in SeeDB2S. Intensity curves were normalized to the maximum intensity in each condition. Note that the refractive index mismatch increases also refraction of light in the interface between the glass coverslip and the medium and reduces brightness. (D, E) High-resolution confocal imaging of *Thy1-YFP-H* mouse brain slices (cerebral cortex) using an oil-immersion objective lens (100×, NA 1.40, WD 0.13 mm). Samples in PBS (D) and SeeDB2S (E) are shown. SeeDB2S extend the imaging depth possible with the high-NA objective lens. (F) Axonal convergence of olfactory sensory neurons expressing transgenic odorant receptor 17 labelled with *IRES-ECFP* (Reference Literature: Imai et al. (2006). Science 314, 657-661). An olfactory bulb sample (2 month old) was cleared with SeeDB2G and imaged with a 63× glycerol-immersion objective lens (NA 1.30, WD 0.30 mm). Z-stacked images (262 µm thick, 3 × 3 tiles) are shown. (G) Hippocampal CA1 region (238 µm × 127 µm × 130 µm) of a *Thy1-YFP-H* mouse brain slice was imaged using a 100× oil-immersion objective lens (NA 1.40, WD 0.13 mm). Both lateral resolution and axial resolutions were sufficiently high to trace individual EYFP-labelled neurons. (H) Reconstructions of four pyramidal neurons from the 3D image of (G) of Fig. 3. Using semi-automated tracing software, Neurolucida, the inventors of the present invention could obtain near-complete wiring patterns from this densely labelled brain slice. Mice were P48-56. Scale bars represent 200 nm in (A), 50 µm in (D, left), (E, left), (F), and 2 µm in (D, right), (E, right).

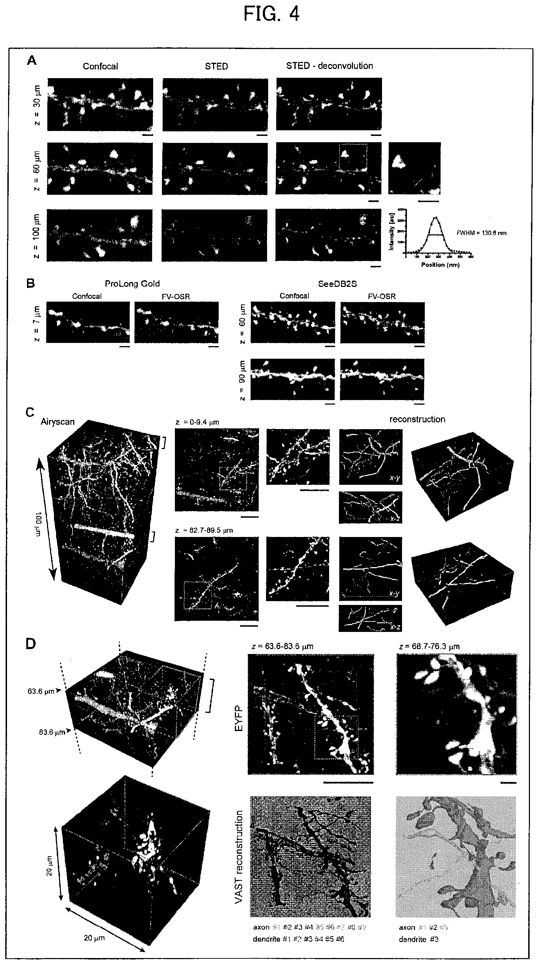

Fig. 4 is a view illustrating super-resolution imaging of dendritic spines in an adult mouse brain slice cleared with SeeDB2S. (A) STED microscopy of *Thy1-YFP-H* mouse brain slices cleared with SeeDB2S. Neural spines of cortical pyramidal neurons are shown (z-stacked images). Confocal images and STED images are compared. STED images were further deconvoluted. Arrangements of spine necks which overcome the classical diffraction limit are well resolved by STED microscopy. Representative FWHM of spine necks (dotted lines) are shown on the right. (B) Dendritic spines of cortical pyramidal neurons were performed with use of an FV-OSR super-resolution system (z-stack). An oil-immersion objective lens (100×, NA 1.40, WD 0.13 mm) was used. In FV-OSR, a sample cleared with SeeDB2S can be improved in resolution but a sample cleared with ProLong Gold cannot be cleared. This means that logical PSF needs to be maintained in order to obtain a super-resolution image. (C) Fine structures of dendritic spines and axonal boutons were imaged with an Airyscan super-resolution system (*Thy1-YFP-H* mouse cerebral cortex, z-stack). Oil-immersion objective lens (100×, NA 1.46, WD 0.13 mm). Volume rendering is shown on the left. Reconstruction is shown on the right. (D) 3D rendering and VAST reconstruction for a part of (C). Scale bars are 1 µm in (A), (B), (D, right), and 10 µm in (C), (D, left).

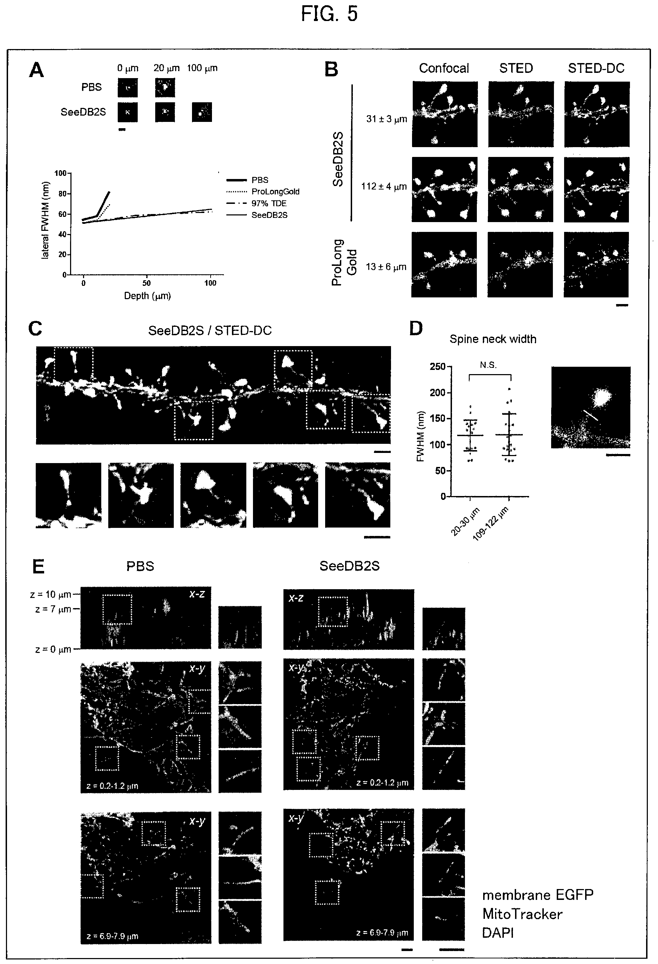

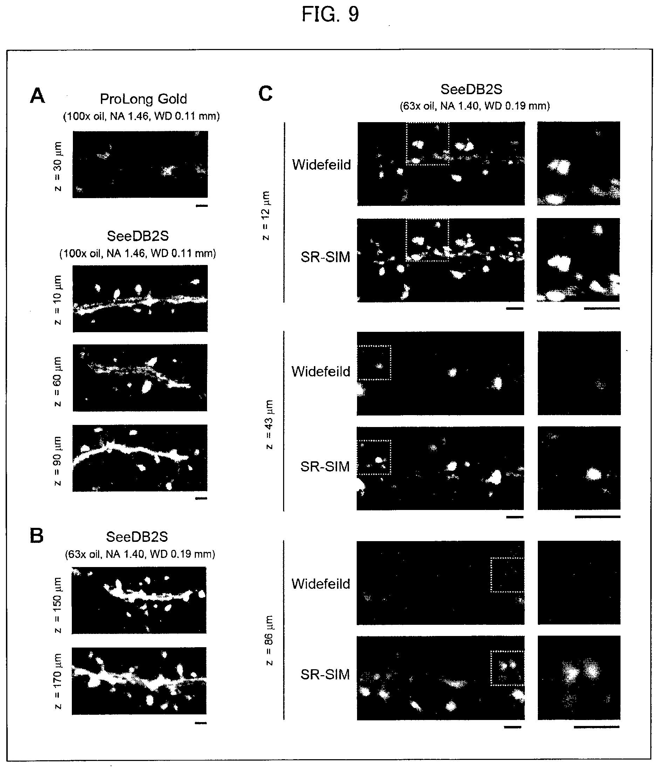

Fig. 5 is a view illustrating deep-tissue super-resolution imaging using SeeDB2S. (A) PSF analysis of fluorescent microspheres (diameter, 40 nm) in various mounting media was determined using STED microscopy with an oil-immersion lens (100×, NA 1.46, WD 0.13 mm). Lateral FWHM obtained with 2D STED is shown. n = 20 each. (B) STED microscopy of the *Thy1-YFP-H* mouse brain slices cleared with SeeDB2S. Dendritic spines of cortical pyramidal neurons are shown (z-stacked images). Confocal and STED images are compared. STED images were further deconvoluted (DC) on the right. Fine spine geometry was fully resolved using STED microscopy up to ∼100 µm depth in SeeDB. In contrast, super-resolution images could not be obtained with ProLong Gold even at ∼10 µm depth. (C) Representative STED images cortical pyramidal neurons (∼60 µm depth). Arrowheads indicate filopodia extending from spine heads. (D) To evaluate resolution of STED microscopy at surface (20-30 pm) and deep (109-122 pm) areas, the inventors of the present invention quantified spine neck diameter, which is known to be below the diffraction limit. A lateral FWHM of spine neck diameter was comparable between two depths (∼120 nm, data are mean ± SD; N.S., non-significant in Welch's test, n = 20 each), suggesting consistent resolution of the STED microscopy. Mice were P56-64. (E) HEK293T cells expressing membrane EGFP were stained with DAPI (nuclei) and MitoTracker (mitochondria) and imaged using 3D SR-SIM. Optical x-z or x-y sections (1 µm stacked images) are shown. An oil-immersion objective lens (63×, NA = 1.40) was used. Therefore, z-axis scales were expanded in PBS samples with RI 1.33. Calibrated depths are shown in the figures. Note that fine filopodia extending from the plasma membrane were better resolved in samples cleared with SeeDB2S than in PBS. Due to quenching of EGFP signals, the inventors of the present invention could not obtain serial SR-SIM images in samples cleared with ProLong Gold and TDE. SR-SIM images of mouse brain slices are shown in C of Fig. 9. Scale bars are 100 nm in (A), 1 µm in (B)-(D), and 2 µm in (E).
Fig. 6 is a view illustrating optimization of SeeDB2. (A) Principles of SeeDB2. In high-resolution imaging, light scattering and spherical aberration reduce the resolution of images in deep area. When samples are cleared with SeeDB2S, refractive indices of immersion oil, glass coverslip, and samples become consistent (1.518). This enables aberration-free high-resolution imaging. (B) Chemical structure of iohexol, a non-ionic triiodobenzene used as an X-ray contrast medium. (C) Refractive indices of iohexol solution at various concentrations. Note that the concentration is indicated by % (w/w). (D) Transmission curves of clearing agents in visual and IR range. (E) Transmission curves of cleared brain samples (postnatal day (P) 21-24, cerebral cortex) in visual and IR range. (F) Effects of various detergents in facilitating clearing by iohexol. Saponin was the most effective; in contrast, Tween 20, previously used in RIMS did not improve clearing. (G) Effects of buffers in improving transparency of brain samples. Tris-EDTA buffer facilitated clearing performance, especially at RI 1.518. For RI 1.518, transmittance was quantified just after clearing (day 3) and after 2 weeks storage. Brain samples cleared with SeeDB2S without buffers could not maintain transparency after long-term storage. n = 3 each.
Fig. 7 is a view illustrating brightness and resolution in SeeDB2. (A) Fluorescence spectra of four Alexa dyes in various clearing media. Fluorescence levels were normalized to the maximum intensity in PBS. Excitation and emission wavelength are indicated in each panel. Note that these dyes were relatively stable in SeeDB2, compared to 97% TDE, a commonly used mounting medium for super-resolution microscopy. Fluorescence of Alexa 555 was increased in the three clearing/ mounting media. The inventors of the present invention found a spectral shift of Alexa 647 in SeeDB2S and TDE. n = 3 each. (B) Autofluorescence signals in wild-type mouse brain slices after incubation in SeeDB or SeeDB2G for 7 days. Images of the cerebral cortex were taken under the same conditions. Autofluorescence accumulates in SeeDB, but not in SeeDB2. (C) Fluorescence of EYFP signals in the *Thy1-YFP-G* mouse brain samples cleared with SeeDB2G (day 0 and day 50, stored at room temperature). Z-stacked images (20 pm) of mitral cells are shown. Imaging conditions were same between these images. (D) Time course of fluorescence changes during clearing with SeeDB2G (room temperature) and CUBIC (37°C) (n = 14 ROI each). Cryosections of Thy1-YFP-G line mice were analyzed. Fluorescence level was gradually reduced in CUBIC, but not in SeeDB2G. (E) Fluorescence spectra of fluorescent proteins after preservation in SeeDB2S for 45 days at room temperature. n = 3 each.

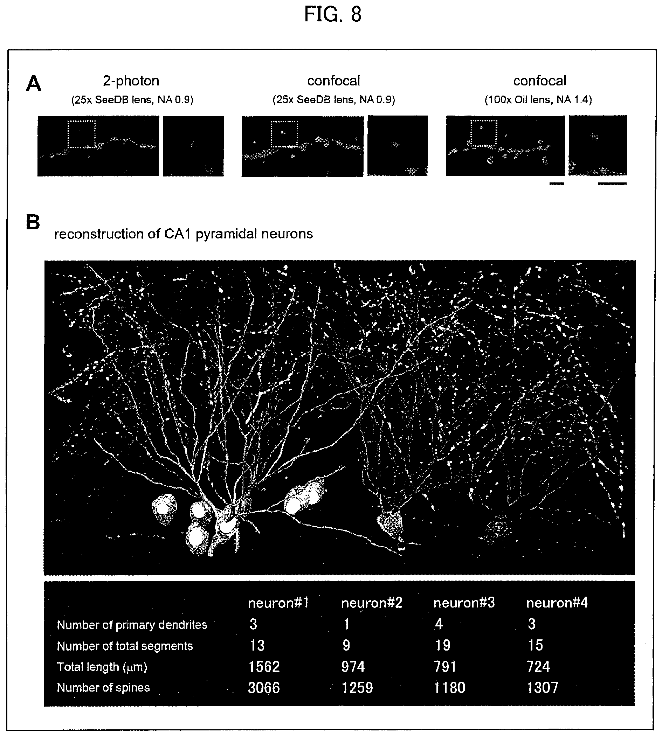

Fig. 8 is a view illustrating confocal imaging of pyramidal neurons in hippocampal CA1 region. (A) Fluorescence images of dendritic spines in *Thy1-YFP-H* mouse brain slices (cerebral cortex, cleared with SeeDB2S). Highest resolution was obtained with confocal imaging using high NA objective lens, consistent with theoretical prediction (d = 0.61λ/NA). Scale bars are 2 µm. (B) High-magnification images of reconstructions shown in G of Fig. 3 (Hippocampal CA1 pyramidal neurons). Quantification of dendritic branches and spines are shown.
Fig. 9 is a view illustrating super-resolution imaging of cortical pyramidal neurons. (A) Super-resolution images (2 µm, z-stack) obtained with Airyscan and a 100× oil-immersion objective lens (NA = 1.46, WD = 0.13 mm). The inventors of the present invention analyzed dendritic spines of cortical pyramidal neurons in *Thy1-YFP-H* mouse brain slices cleared with SeeDB2S. Note that the inventors of the present invention could not obtain super-resolution images for samples mounted in ProLong Gold. (B) Super-resolution images (z-stacked) obtained with Airyscan and a 63× oil-immersion objective lens (NA = 1.40, WD = 0.19 mm). (C) Super-resolution images (2-9 µm z-stack) obtained with SR-SIM. The inventors of the present invention obtained well-resolved images up to ∼40 µm using mouse cortical slices. Scale bars represent 1 µm.

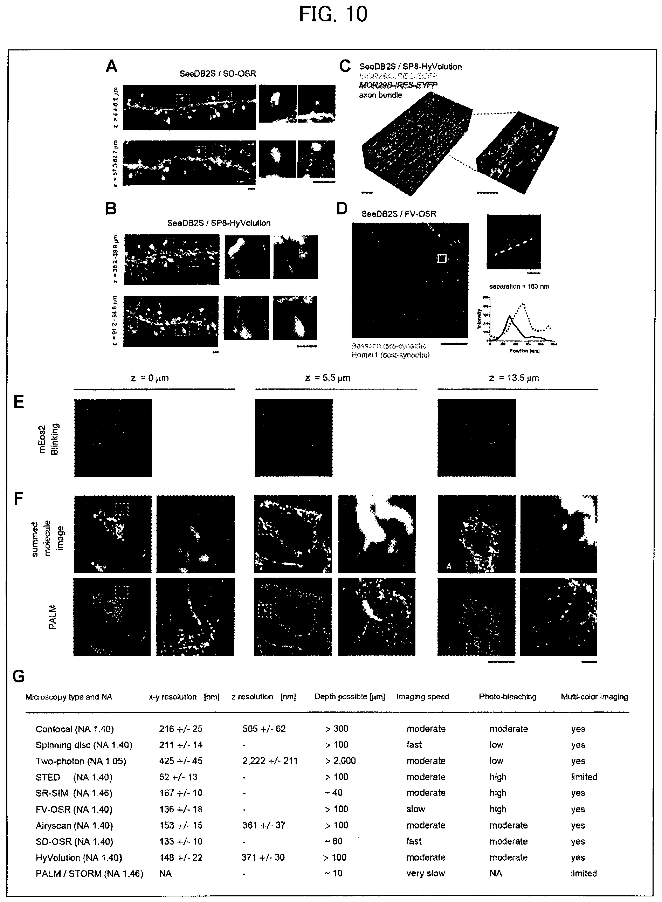

Fig. 10: (A) Super-resolution images obtained with SD-OSR. (B) Super-resolution images obtained with SP8 HyVolution. (C) Multi-color super-resolution images of axons for olfactory sensory neurons obtained with SP8-Hyvolution. (D) A mouse brain slice (cerebral cortex) immunostained with anti-Bassoon (pre-synaptic marker) and anti-Homerl (post-synaptic marker) was imaged with FV-OSR. Depth was 2.45-3.50 µm. These two proteins were separated in the super-resolution images as reported previously (Dani, A., et al. (2010). Neuron 68, 843-856). (E) A snapshot of blinking images of LifeAct-mEos2 for PALM imaging. HEK293T cells expressing LifeAct-mEos2 were imaged. (F) LifeAct-mEos2 imaged with PALM. Super-resolution images were reconstructed from 10,000 images. Note that actin filament is well resolved by PALM. (G) Utility of various microscopies tested for SeeDB2. Scale bars are 1 µm in (A) and (B), 5 µm in (D, left), and 200 nm in (E, right), 10 µm in (C), (F, left), and 1 µm in (F, right).

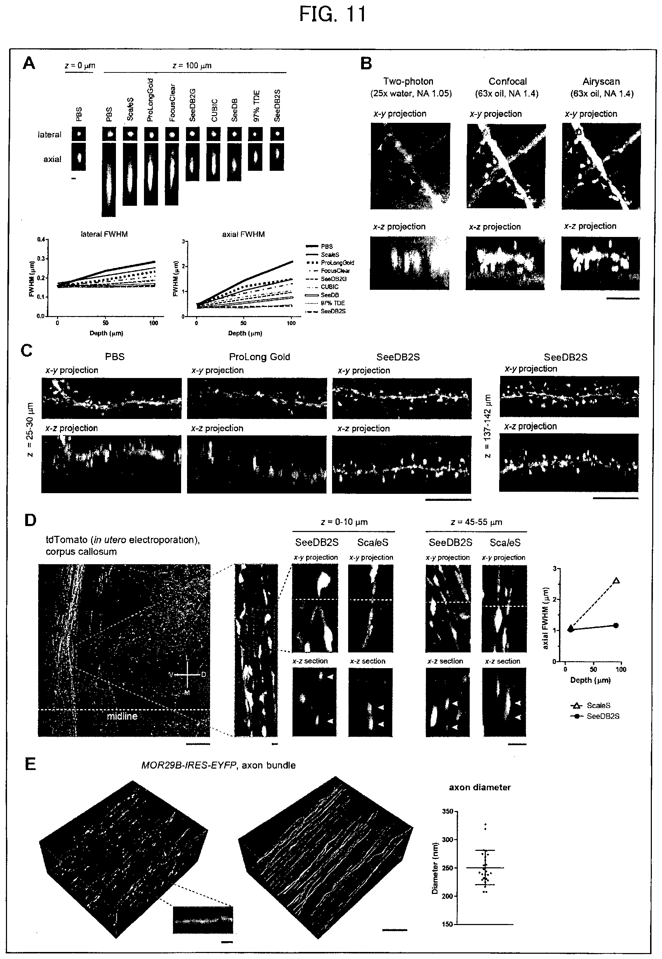

Fig. 11 is a view illustrating improved performance of neuronal tracing with SeeDB2S. (A) Point-spread-function (PSF) analysis of fluorescent microspheres (diameter, 100 nm) imaged with Airyscan super-resolution microscopy. FWHM for lateral and axial directions are shown. n = 20 each. (B) Comparison of resolution obtained with two-photon microscopy, confocal microscopy, and Airyscan microscopy (*Thy1-YFP-H,* cerebral cortex, P48-64). The "stubby" spines found in two-photon microscopy were not "stubby" in Airyscan (two arrowheads on the left). Some spines detected in Airyscan were missing in two-photon microscopy (two arrowheads on the right). (C) Brain slices from adult *Thy1-YFP-H* mouse (P181) in PBS, ProLong Gold and SeeDB2S were imaged with Airyscan super-resolution microscopy. X-y and x-z projection images of dendrites are shown. Note that axial resolution is much improved with SeeDB2S. (D) Tracing densely-labelled axons in the corpus callosum (Airyscan). Layer 2/3 pyramidal neurons were labelled with tdTomato using in utero electroporation. X-z sections of crossing-over points are shown in lower boxes. Axial FWHM of axon signals are mean ± SEM. D, dorsal; V, ventral; L, lateral; M, medial. (E) Tracing and quantification of axons for olfactory sensory neurons (Airyscan). Axon diameter data are mean ± SD. Dorsal olfactory epithelium and axon bundles were dissected from transgenic *MOR29B-IRES-EYFP* mice and cleared with SeeDB2S. All labelled axons were reconstructed on the right. Scale bars are 200 nm in (A), 5 µm in (B), 10 µm in (C), 100 µm in (D, left), 2 µm in (D, middle, right), 1 µm in (E, left), and 10 µm in (E, right).

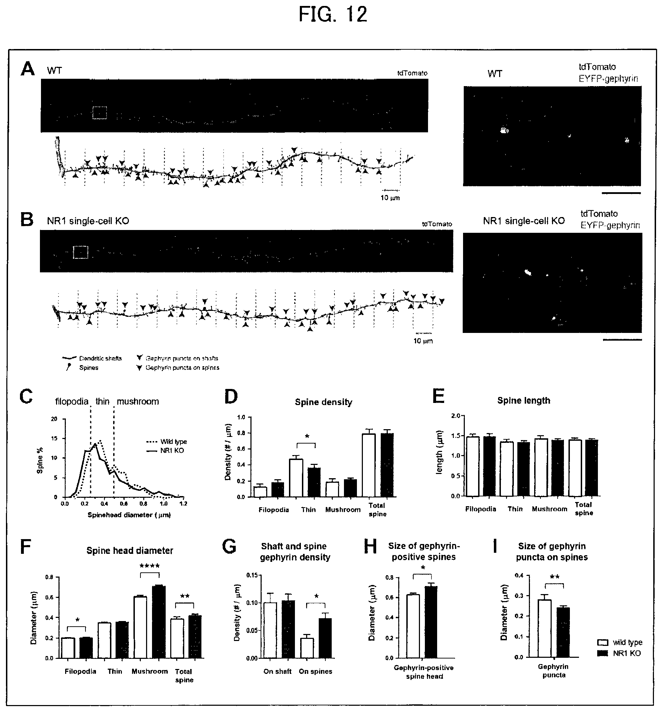

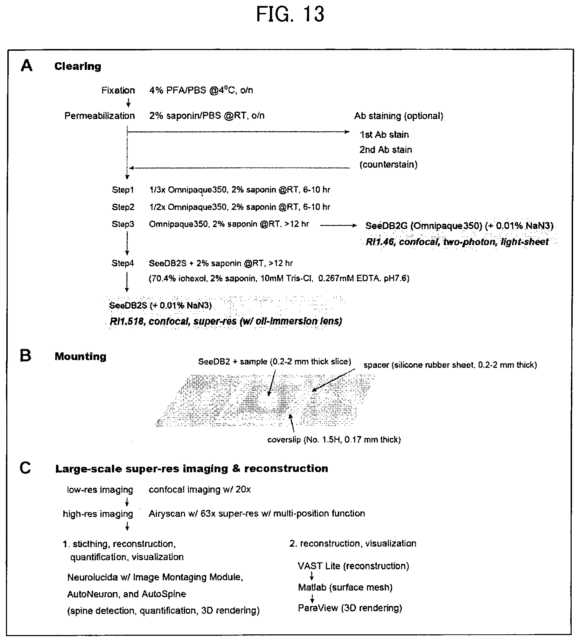

Fig. 12 is a view illustrating volumetric quantitative analyses of excitatory and inhibitory synapses in wild-type and NMDAR-deficient neurons using super-resolution microscopy. (A, B) L5 neurons were labelled with cytoplasmic tdTomato and EYFP-Gephyrin using in utero electroporation and analyzed at P22 (Airyscan). Unbranched oblique dendrites (100-200 µm long) extending from the trunk of the proximal part of apical dendrites of L5 pyramidal neuron were analyzed. Representative super-resolution images are reconstructed with VAST Lite and visualized with ParaView (See Fig. 13 for the analysis pipeline). Note that fine structures of dendritic spines are clearly resolved and gephryin puncta on dendrite shaft vs. spines are unambiguously defined. Both wild-type neurons (A) and NR1-deficient neurons (B) were analyzed. (C) Definition of three types of spines (filopodia, thin, mushroom) based on spine head sizes. Quantifications of spine head density (D), spine length (E), and spine diameter (E) of WT and NMDAR-KO neurons. (G) Density of EYFP-Gephyrin puncta in WT and NMDAR-KO neurons. (H) Sizes of gephyrin-positive spine heads. (I) Size of gephyrin puncta on dendritic spines. *P < 0.05, **P < 0.01, ****P < 0.0001 (Mann-Whitney U test). N = 503 and 1068 spines in (D) and (E). N = 5 (WT) and 8 dendrites (KO) in (F) and (G). N = 22 (WT) and 97 (KO) spines in (H) and (I). Data are mean ± SEM. Scale bars are 2 µm.
Fig. 13 is a view illustrating clearing and analysis pipeline for super-resolution connectomics. (A) Schematic representation of clearing protocol. (B) A mounting procedure for mouse brain slices. (C) Imaging, reconstruction, and quantification pipeline for analyzing neuronal circuitry.

### Description of Embodiments

As a result of diligent study, the inventors of the present application have found that a biological material can be cleared by using a solution containing a detergent and a non-ionic organoiodine compound; morphology of the biological material is well maintained in that case; fluorescence intensity of a fluorescent substance is maintained; and the like. Further, the inventors of the present application have also found that, in a case where a solution containing a non-ionic organoiodine compound is used as a mounting agent for a microscope, it is possible to perform super-resolution imaging up to a deep part under a microscope while maintaining such a state, and the like. Based on those findings, the inventors of the present application arrived at the present invention.

### [1. Method for clearing biological material]

### (Overview)

The method in accordance with an aspect of the present invention for clearing a biological material includes a step (hereinafter, referred to as "clearing step A") of clearing the biological material by immersing the biological material in a solution (hereinafter, referred to as "clearing agent A") which contains a detergent and a non-ionic organoiodine compound. The method in accordance with an aspect of the present invention for clearing a biological material may further include a step (hereinafter, referred to as "observing step A") of observing the biological material, which has been cleared, under a microscope in a state in which the biological material is immersed in a solution (hereinafter, referred to as "mounting agent") that contains a non-ionic organoiodine compound and that has a refractive index higher than 1.48. Moreover, the method in accordance with an aspect of the present invention for clearing a biological material may preferably include a step (hereinafter, referred to as "clearing step B") of clearing the biological material before the clearing step A by immersing the biological material in at least one solution (hereinafter, referred to as "clearing agent B") that contains a detergent and a non-ionic organoiodine compound whose concentration is lower than that of the clearing agent A. In the clearing step B, in a case where two or more solutions are used each of which contains a detergent and a non-ionic organoiodine compound whose concentration is lower than that of the clearing agent A, it is preferable that the biological material is sequentially immersed in the two or more solutions in ascending order of concentration of the non-ionic organoiodine compound.

### (Clearing agent)

The clearing agent A and the clearing agent B are described below while being collectively referred to as "clearing agent".

The clearing agent is a solution containing a detergent and a non-ionic organoiodine compound.

Examples of the detergent encompass saponin, Triton X-100 (Registered Trademark), Nonidet P-40 (product name), and the like. From the viewpoint of high clearing effect, reduction in deformation of biological materials, reduction in loss of fluorescence of fluorescent substances (fluorescent proteins or fluorescent chemical substances), and the like, the detergent is preferably at least one selected from the group consisting of saponin, Triton X-100, and Nonidet P-40, more preferably at least one selected from the group consisting of saponin and Triton X-100. From the viewpoint of high clearing effect and reduction in loss of fluorescence of fluorescent substances, the detergent is even more preferably saponin. It is preferable that the detergent is not Tween 20. It is preferable that the detergent is not SDS. Triton X-100 can be particularly suitably used for relatively small biological materials (e.g., brains of Drosophila, cultured cells, and the like). Saponin is a glycoside of triterpene or steroid. In regard to the clearing agent in accordance with an aspect of the present invention, saponin is not limited to a particular kind. Examples of saponin encompass Quillajaceae derived saponin, soybean derived saponin, karaya gum derived saponin, digitalis derived saponin (digitonin), and the like. A suitable example of saponin can be Quillaja sapponaria (Quillajaceae) derived saponin.

Examples of the non-ionic organoiodine compound encompass a monomer-type non-ionic organoiodine compound having a triiodobenzene ring, a dimer-type non-ionic organoiodine compound having a triiodobenzene ring, and the like. The non-ionic organoiodine compound is preferably a monomer-type non-ionic organoiodine compound. More specific examples of the non-ionic organoiodine compound encompass monomer-type non-ionic triiodobenzene compounds such as iohexol, iopamidol, iopentol, iobitriol, iopromide, iomeprol, ioversol, and ioxilan; dimer-type non-ionic triiodobenzene compounds such as iotrolan and iodixanol; and the like. From the viewpoint of high clearing effect and low consistency, the non-ionic organoiodine compound is preferably a monomer-type non-ionic triiodobenzene compound. Among those, iohexol is preferable because iohexol is inexpensive and is easily available.

The solvent is not limited to a particular kind, provided that the non-ionic organoiodine compound can be solved in the solvent. It is preferable to use water as a main solvent, and it is particularly preferable that only water is used as the solvent. The clearing agent A is preferably an aqueous solution, and the clearing agent B, which is optionally used, is also preferably an aqueous solution. It is more preferable that the clearing agent A and the clearing agent B which is optionally used are both aqueous solutions. Note that, in the present invention, the phrase "use water as a main solvent" means that a volume ratio of water relative to all used solvents is higher than the other solvents, and preferably means that the volume ratio of used water is higher than 50% and 100% or lower relative to a total volume of all used solvents. Moreover, in the present invention, the "aqueous solution" indicates a solution in which water is used as a main solvent.

Main advantageous points to use water as a solvent are as follows: 1) As compared with a case where an organic solvent is used as a main solvent, dehydration does not occur in a biological material to be processed. Therefore, it is possible to inhibit a problem of shrinkage of the biological material. 2) As compared with a case where an organic solvent is used as a main solvent, a possibility of damaging a fluorescent protein notably decreases. Therefore, it is possible to observe a processed biological material with use of a fluorescent protein. 3) It is possible to use the solvent not only for a fixed material but also for a living material. 4) As later described, a clearing process becomes reversible, and it is possible to optionally restore a biological material, which has been subjected to a clearing process, to a state before the clearing process. 5) As compared with a case where an organic solvent is used as a main solvent, safety in handling is further heightened.

The clearing agent may contain a buffer which enables maintenance of pH that is suitable for a biological material to be processed. Examples of the buffer encompass Tris buffers (i.e., buffers which contain Tris (tris(hydroxymethyl)aminomethane) and in which pH is adjusted with acid such as hydrochloric acid) such as Tris, Tris-EDTA (which is a buffer composed of Tris and ethylenediaminetetraacetic acid), and Tris buffered saline (TBS); phosphate buffers such as phosphate buffered saline (PBS) and Hank's balanced salt solution (HBSS) (i.e., buffers containing phosphate); hydroxyethyl piperazine ethanesulfonic acid (HEPES); and the like. Among those, the buffer is preferably Tris buffer. The concentration of Tris contained in the Tris buffer can be, for example, 2 mM to 40 mM, preferably 5 mM to 20 mM, more preferably approximately 10 mM. From the viewpoint of maintaining transparency of a biological material, a concentration of salt in the buffer is preferably low. In a case where the clearing agent has a buffering ability, it is possible to further prevent breakage of fragile biological materials and fluorescence attenuation of fluorescent substances. Moreover, in a case where the clearing agent contains a buffer, it is possible to more effectively maintain a cleared state. The pH of the buffer can be set as appropriate according to the type or purpose of a biological material. For example, the pH of the buffer is preferably 7 to 8, and more preferably 7.4 to 7.6. Note that the buffer can be an aqueous solution, and the clearing agent can be an aqueous solution.

The clearing agent may optionally contain an additive. Examples of the additive encompass a preservative such as sodium azide, an anti-fluorescence-quenching agent such as DABCO, DAPI for nuclear staining, and low-melting agarose for maintaining morphology of samples.

2,2'-thiodiethanol (TDE) has the ability to quench fluorescence. Therefore, in one embodiment, the clearing agent contains no TDE.

### (Clearing step A)

The clearing step A is a step of clearing a biological material by immersing the biological material in the clearing agent A. Specifically, for example, the biological material is immersed in the clearing agent A in a container for clearing process.

In the clearing step A, the biological material is cleared. Here, the term "cleared" means to be transparent as compared with a biological material which has not been subjected to the method (or clearing step) in accordance with an aspect of the present invention for clearing a biological material yet and, preferably means to allow light having a wavelength of 400 µm to 1300 µm to further pass through, as compared with a biological material which has not been subjected to the method (or clearing step) in accordance with an aspect of the present invention for clearing a biological material yet.

The concentration of the detergent contained in the clearing agent A is not particularly limited. In a case where a biological material is relatively thick (e.g., a fetus of mouse, a brain of mouse, or the like), the concentration of the detergent is preferably within a range from 0.01% (w/v) to 20% (w/v), more preferably within a range from 0.5% (w/v) to 5% (w/v), even more preferably 2% (w/v). In a case where a biological material is relatively thin (e.g., a cultured cell), the concentration of the detergent is preferably lower than the above exemplified concentrations.

The concentration of the non-ionic organoiodine compound contained in the clearing agent A is not particularly limited, and is preferably a concentration that achieves a refractive index near the refractive index of an immersion liquid for use in microscopy, more preferably a concentration that achieves a refractive index equal to the refractive index of the immersion liquid. The refractive index of the clearing agent A is, for example, 1.46 or higher, higher than 1.48, 1.49 or higher, 1.50 or higher, or 1.51 or higher, and 1.70 or lower, 1.65 or lower, 1.60 or lower, 1.55 or lower, 1.54 or lower, 1.53 or lower, or 1.52 or lower. In a more preferable example, the refractive index of the clearing agent A is approximately 1.46 or approximately 1.518. In a further preferable example, the refractive index of the clearing agent A is approximately 1.518. For example, in a case where an immersion liquid (immersion oil) having a refractive index of 1.518 is used in microscopy, the concentration of the non-ionic organoiodine compound (in particular, iohexol) is preferably within a range from 30% (w/w) to 90% (w/w), more preferably within a range from 40% (w/w) to 80% (w/w), even more preferably 70.4% (w/w). Note that the concentration of the non-ionic organoiodine compound in the clearing agent A having an intended refractive index can be calculated with reference to, for example, C of Fig. 6.

Note that the unit "% (w/v)" is a percentage of a weight (w (gram)) of a solute (detergent, non-ionic organoiodine compound, or the like) relative to a volume (v (milliliter)) of a solution (clearing agent or the like). The unit "% (w/w)" is a percentage of a weight (w (gram)) of a solute relative to a weight (w (gram)) of a solution.

The temperature at which the clearing step A is performed is not particularly limited, and is preferably within a range of 0°C or above and 50°C or below, from the viewpoint of maintaining fine morphology and a fluorescent dye. The time for which the clearing step A is performed is not particularly limited, and may be selected appropriately according to the type or thickness of the biological material. For example, the time is preferably within a range of 1 hour or longer and 90 days or shorter, and more preferably within a range of 2 hours or longer and 10 days or shorter. The pressure at which the clearing step A is performed is not particularly limited, and may be selected appropriately according to the origin or the purpose of the biological material. The pressure may be, for example, atmospheric pressure.

### (Clearing step B)

The clearing step B which is optionally performed is a step of clearing, before the clearing step A, the biological material by immersing the biological material in at least one clearing agent B. In a case where two or more clearing agents B are used in the clearing step B, it is preferable that the biological material is sequentially immersed in the two or more clearing agents B in ascending order of concentration of the non-ionic organoiodine compound. Specifically, for example, the biological material is sequentially immersed in the at least one clearing agent B in a container for clearing process in ascending order of concentration of the non-ionic organoiodine compound.

In the clearing step B, the biological material is cleared. Note, however, that achieved transparency is lower than that in the clearing step A. As the concentration of the non-ionic organoiodine compound increases, transparency becomes higher. That is, in a case where the clearing step B is performed, the concentration of the non-ionic organoiodine compound is gradually increased from the clearing step B to the clearing step A, and thus the transparency of the biological material is gradually heightened. In a case where the clearing step B is performed before the clearing step A, it is possible to inhibit sharp rise of the concentration of the non-ionic organoiodine compound, and can thus further inhibit damage on the biological material.

The concentration of the non-ionic organoiodine compound contained in each clearing agent B is not limited in particular, provided that the concentration is lower than the concentration of the non-ionic organoiodine compound contained in the clearing agent A. In one embodiment, it is preferable to use two or more clearing agents B having different concentrations, and it is more preferable to use three or more clearing agents B having different concentrations. This is because, by raising the concentration more minutely in stages, it is possible to further inhibit damage on the biological material.

In a case where an immersion oil having a refractive index of 1.518 is used in microscopy, for example, it is possible to use, as the clearing agents B, three solutions in which concentrations of the non-ionic organoiodine compound (in particular, iohexol) are 18.7% (w/w), 28.1% (w/w), and 56.2% (w/w), respectively. In this case, in the subsequent clearing step A, it is preferable to use, as the clearing agent A, a solution in which a concentration of the non-ionic organoiodine compound (in particular, iohexol) is 70.4% (w/w).

From the viewpoint of maintaining refractive index matching, it is preferable that the non-ionic organoiodine compound in each of the clearing agents B is of the same kind as that in the clearing agent A.

To the type and concentration of the detergent in each of the clearing agents B, the descriptions of the type and concentration of the buffer are applicable. To the temperature at which the clearing step B is performed, the description of the temperature in the clearing step A is applicable. The type and concentration of the detergent in each of the clearing agent B and the type and concentration of the buffer in each of the clearing agent B are preferably identical with those in the clearing agent A. Further, the temperature at which the clearing step B is performed is preferably identical with that in the clearing step A. In a case where two or more clearing agents B are used, processing times in the respective clearing agents B having different concentrations are not particularly limited. As an example, it is possible to perform processes for 6 hours to 10 hours in the clearing agent B whose concentration of non-ionic organoiodine compound is 18.7% (w/w), for 6 hours to 10 hours in the clearing agent B whose concentration of non-ionic organoiodine compound is 28.1% (w/w), and for 8 hours to 12 hours in the clearing agent B whose concentration of non-ionic organoiodine compound is 56.2% (w/w). The pressure at which the clearing step B is performed is not particularly limited, and may be selected appropriately according to the origin or the purpose of the biological material. The pressure may be, for example, atmospheric pressure.

In the clearing step A and the clearing step B, an order of putting the "clearing agent" and the "biological material" into the container for clearing process is not particularly limited. In an example in which the clearing step A and the clearing step B are continuously performed in the same container, first, a "clearing agent B" whose concentration of the non-ionic organoiodine compound is lowest is put into the container, and then the "biological material" is put into the container. Next, the clearing agent B of the lowest concentration is discarded, and a clearing agent B whose concentration of the non-ionic organoiodine compound is second lowest is put into the container. Then, the clearing agent B of the second lowest concentration is discarded, and a clearing agent B whose concentration of the non-ionic organoiodine compound is third lowest is put into the container. These sorts of processes are repeatedly performed, and a clearing agent B having a highest concentration of the non-ionic organoiodine compound is put into the container and is then discarded. Lastly, the clearing agent A (whose concentration of the non-ionic organoiodine compound is higher than that of the clearing agent B of the highest concentration) is put into the container, and is then discarded. In order to enhance permeability of the clearing agent, the biological material in the container may be mixed with use of a shaker or a rotator. Moreover, it is possible to optionally provide a step of performing washing treatment or the like with respect to the container and/or the biological material in between replacement of clearing agents of different concentrations.

The treatment container which has been used in the clearing steps and contains the biological material subjected to the clearing process may be preserved in, for example, an environment at a room temperature or a low temperature (cleared sample preserving step) until the biological material is subjected to an observing step which will be described later. In the treatment container, the biological material can be in a state in which the biological material is immersed in the clearing agent A or the clearing agent B.

### (Mounting agent)

The mounting agent is a solution that contains a non-ionic organoiodine compound and that has a refractive index higher than 1.48.

To the type of non-ionic organoiodine compound in the mounting agent, the descriptions in the (Clearing agent) section are applicable. From the viewpoint of maintaining refractive index matching, it is preferable that the non-ionic organoiodine compound in the mounting agent is of the same kind as that in the clearing agent A.

The concentration of the non-ionic organoiodine compound in the mounting agent is not particularly limited, and is preferably a concentration that achieves a refractive index near the refractive index of an immersion liquid for use in microscopy, more preferably a concentration that achieves a refractive index equal to the refractive index of the immersion liquid. For example, in a case where an immersion oil having a refractive index of 1.518 is used in microscopy, the concentration of the non-ionic organoiodine compound (in particular, iohexol) is preferably within a range from 30% (w/w) to 90% (w/w), more preferably within a range from 40% (w/w) to 80% (w/w), even more preferably 70.4% (w/w). Note that the concentration of the non-ionic organoiodine compound in the mounting agent having an intended refractive index can be calculated with reference to, for example, C of Fig. 6.

The solvent is not limited to a particular kind, provided that the solvent can dissolve the non-ionic organoiodine compound. It is preferable to use water as a main solvent, and it is particularly preferable to use water alone as the solvent. The main advantages of using water as the solvent have already been described.

The mounting agent may contain a buffer that can keep the pH suitable for the to-be-observed biological material. To the buffer, the descriptions in the (Clearing agent) section are applicable. It is noted that the buffer may be an aqueous solution and that the mounting agent may be an aqueous solution. Especially in a case where the mounting agent contains the non-ionic organoiodine compound at high concentration, the buffer in the mounting agent is preferably of the same kind as that of the clearing agent A in order to keep transparency.

The mounting agent may optionally contain some additive(s). Additives are added for the purpose of, for example, long-term storage, prevention of color fading, nuclear staining, preservation of sample morphology, and/or the like. Examples of the additives include sodium azide, low-melting agarose, antifade chemicals, and DAPI. The concentration of the additive(s) is not particularly limited, and may be selected appropriately according to its purpose. The concentration may be, for example, 0.1% (w/v) to 5% (w/v).

The mounting agent may or may not contain any of the earlier-described detergents. That is, the mounting agent may be a solution that contains a non-ionic organoiodine compound but contains no detergents. The mounting agent without detergents is advantageous in that little foaming occurs.

The refractive index of the mounting agent is preferably as close as possible to the refractive index of an immersion liquid for use in microscopy. The refractive index of the mounting agent depends on the refractive index of the non-ionic organoiodine compound per se and the concentration of the non-ionic organoiodine compound. The refractive index of the mounting agent is, for example, higher than 1.48, 1.49 or higher, 1.50 or higher, or 1.51 or higher, and, 1.70 or lower, 1.65 or lower, 1.60 or lower, 1.55 or lower, 1.54 or lower, 1.53 or lower, or 1.52 or lower. It is more preferable that the refractive index of the mounting agent be about 1.518. Especially in a case where the mounting agent contains the non-ionic organoiodine compound at high concentration, the non-ionic organoiodine compound in the mounting agent is preferably substantially the same in concentration as that of the clearing agent A in order to keep transparency.

2,2'-thiodiethanol (TDE) has the ability to quench fluorescence. Therefore, in one embodiment, the mounting agent contains no TDE.

### (Observing step A)

The observing step A is a step of observing, under a microscope, a cleared biological material in a mounting agent. The observing step A is performed after the clearing step A. More specifically, for example, a biological material, which has undergone the clearing step A, is immersed in a mounting agent in a container and gently inverted to mix and equilibrate, and then the biological material is immersed in a mounting agent in another container to equilibrate. The biological material in the mounting agent is sealed with a glass coverslip for microscopy.

The microscope is not limited to a particular kind. Examples of the microscope include: optical microscopes such as confocal laser microscopes, light-sheet microscopes, multi-photon excitation microscopes (typically two-photon excitation microscope), STED microscopes, RESOLFT microscopes, SIM microscopes, PALM/STORM microscopes, Bessel beam microscopes, and lattice light-sheet microscopes; and high-resolution imaging using such a microscope and image processing (deconvolution) in combination. Microscopes with oil-immersion lenses (or oil-immersion objective lenses) may be used. In the clearing method in accordance with an aspect of the present invention, the refractive index of the mounting agent can be adjusted to 1.518, and quenching of fluorescent proteins and fluorescent chemical substances and fine morphological changes of biological materials can be reduced. Therefore, the clearing method in accordance with an aspect of the present invention can be suitably used particularly in high-resolution imaging or super-resolution microscopy which use an immersion oil having a refractive index of 1.518 and which are recently under development. The term "high-resolution imaging" denotes imaging using a high-NA (1 or greater) oil-immersion lens, by which a high resolution close to the light diffraction limit is obtained. The term "super-resolution microscope" denotes a microscope which enables observation at a resolution beyond the theoretical limit (about 200 nm in plane direction) of optical microscopy. Examples of super-resolution microscopes include: STED microscopes, SIM microscopes, PALM/STORM microscopes, and Airyscan microscopes.

The temperature at which the microscopy is performed is not particularly limited, and may be within a similar range to that described earlier for the clearing step A.

### (Pre-treatment step)

In one embodiment of the present invention, the method for clearing a biological material may further include, prior to clearing, a step of immersing the biological material in a solution that contains a detergent but contains no non-ionic organoiodine compounds (such a solution is hereinafter referred to as "pre-treatment solution", and such a step is hereinafter referred to as "pre-treatment step"). In a case where the method includes the clearing step B, the pre-treatment step is performed before the clearing step B. In a case where the method does not include the clearing step B, the pre-treatment step is performed before the clearing step A.

Performing the pre-treatment step is advantageous in that this step enables tissue staining such as antibody staining or in-situ hybridization. It is noted that the biological material is not cleared in the pre-treatment step.

To the type and concentration of the detergent in the pre-treatment solution, the description of the clearing agent A is applicable. It is preferable that the type and concentration of the detergent in the pre-treatment solution are the same as those of the clearing agent A.

The solvent in the pre-treatment solution is not limited to a particular kind. It is preferable to use water as a main solvent, and it is particularly preferable to use water alone as the solvent.

The pre-treatment solution may contain a buffer that can keep the pH suitable for the to-be-treated biological material. Examples of the buffer include those listed in the (Clearing agent) section. Of those buffers listed above, PBS is preferred from the viewpoint of keeping properties of proteins. The pH of the buffer may be selected appropriately according to the type or purpose of the biological material. For example, the pH is preferably 3 to 11, more preferably 7 to 8. It is noted that the buffer may be an aqueous solution and that the pre-treatment solution may be an aqueous solution.

The temperature at which the pre-treatment step is performed is not particularly limited, and preferably within a range of 0°C or above and 50°C or below, from the viewpoint of preserving tissue morphology. The amount of time during which the pre-treatment step is performed is not particularly limited, and may be selected appropriately according to the type or thickness of the biological material. For example, the amount of time during which the pre-treatment step is performed is preferably within a range of 1 hour or longer and 30 days or shorter, more preferably within a range of 2 hours or longer and 1 day or shorter. The pressure at which the pre-treatment step is performed is not particularly limited, and may be selected appropriately according to the origin or the purpose of the biological material. The pressure may be, for example, atmospheric pressure.

The pre-treatment step may be performed in, for example, the container for the clearing described earlier.

### (Visualizing step)

The biological material to be subjected to the observing step A may optionally undergo a visualizing step, such as staining or marking, prior to the clearing step A or B or between the clearing step A and the observing step.

For example, in a case where a fluorescent protein is used in the visualizing step, a fluorescent protein gene is introduced into a living biological material and the gene is allowed to express a fluorescent protein, prior to the clearing step A or B. Examples of the fluorescent protein include CFP, YFP, GFP, and tdTomato.

On the other hand, in a case where the visualizing step includes injecting a fluorescent chemical substance (except fluorescent proteins) into a biological material or includes staining a biological material with a fluorescent chemical substance, the visualizing step may be performed after the clearing step A, although it is preferably performed before the clearing step A or B. As an alternative, the visualizing step may include staining with the use of a chemical substance other than fluorescent chemical substances.

According to the clearing method in accordance with an aspect of the present invention, stained areas, marked areas, or the like areas even at deep depth are less likely to lose color. Therefore, it is possible to more clearly observe the stained areas, marked areas, or the like areas in the biological material. In particular, since the clearing method in accordance with an aspect of the present invention is not likely to reduce resolution and enables imaging in deeper areas, the method enables observation of the stained areas, marked areas, or the like areas deep inside the biological material. In one embodiment, it is possible to perform high-resolution fluorescence imaging at depth deeper than ever before.

### (Other steps)

In one embodiment of the present invention, the clearing method may further include some other step(s) in addition to the above-described steps. Such other step is, for example, a step of performing a commonly-used treatment like those listed below for the purpose of, for example, heightening the efficiency of tissue staining or for the purpose of removing lipid:
1) a treatment with ethanol or methanol, or a treatment with a combination of ethanol or methanol with an organic solvent (e.g., treatment with 50% methanol, 80% ethanol, or 100% methanol),
2) freezing/thawing of tissues (e.g., three cycles of freezing with liquid nitrogen followed by thawing), or
3) heat treatment of tissues (e.g., treat tissues in a citrate buffer solution at 120°C for 15 minutes).

When to perform such steps may be selected as appropriate.

In one embodiment of the present invention, the clearing method may include another observing step (observing step A') instead of the observing step A. The observing step A' includes observing, under a microscope, a cleared biological material in a solution that contains a non-ionic organoiodine compound and that has a refractive index of 1.48 or lower. The details of this solution are basically the same as those described for the mounting agent, except the description related to the refractive index. In one example, the refractive index of the solution is 1.46. In this case, the concentration of the non-ionic organoiodine compound (in particular, iohexol) in the clearing agent A is preferably 56.2% (w/w), whereas, in the case of using the clearing agent B, it is preferable that the clearing agent B contains two solutions containing the non-ionic organoiodine compound (in particular, iohexol) at concentrations of 18.7% (w/w) and 28.1% (w/w), respectively.

### (Biological material to be subjected to the method)

There is no particular limitation on what biological material is subjected to the clearing method in accordance with an aspect of the present invention. However, the biological material is preferably derived from a plant or an animal, more preferably derived from an animal such as a fish, an amphibian, a reptile, a bird, or a mammal, particularly preferably derived from a mammal. Mammals are not limited to a particular kind, and examples of mammals include: laboratory animals such as mice, rats, rabbits, guinea pigs, and non-human primates; pets such as dogs and cats; domestic animals such as cattle, horses, and pigs; and humans.

The biological material may be an individual itself (except a living human individual itself) or may be an organ, a tissue, or a cell taken from an individual of a multicellular organism (which may be a human) or artificially cultured from them. For example, a pathological sample for disease diagnosis, which is derived from a human or a non-human animal, may be used as the biological material. The pathological sample may be an organ, a tissue, or a cell. Alternatively, the biological material may be a pluripotent cell such as an iPS cell or an ES cell or may be a cell or a tissue differentiated from a pluripotent cell. The clearing agent for use in an aspect of the present invention has excellent clearing ability and the mounting agent of an aspect of the present invention enables imaging in deeper areas. Therefore, the clearing agent and the mounting agent are applicable regardless of whether the biological material is a tissue or an organ (e.g., whole brain or partial brain) derived from a multicellular animal or whether the biological material is an individual (e.g., embryo) of a multicellular non-human animal itself. The biological material may be a material without lipid removal.

The clearing agent and the mounting agent for use in an aspect of the present invention cause little swelling or shrinkage of biological materials (see also Examples) and have a significant effect of reducing deformation of biological materials, and therefore are particularly suitable for clearing of fragile biological materials. Examples of the fragile biological materials include: brains of newborn mice; tissue sections derived from multicellular animals; animal embryos in early stage of development; brains of small fishes such as zebrafish; brains of invertebrates such as Drosophila; and oocytes.

Furthermore, the clearing agent and the mounting agent for use in an aspect of the present invention are less likely to cause breakage of the fine structures of biological materials and they reduce potential artifacts. Therefore, it is possible to more accurately observe fine structures such as neuronal circuits. The method of an aspect of the present invention is thus suitable for use even in a case where the biological material is a tissue containing a neuronal circuit (e.g., brain).

Alternatively, as described earlier, the biological material may be, for example, a biological tissue injected with a fluorescent chemical substance, a biological tissue stained with a fluorescent chemical substance, a biological tissue into which a fluorescent protein-expressing cell has been transplanted, a biological tissue of an animal genetically modified to express a fluorescent protein, or the like. That is, the biological material may be labelled with a fluorescent protein or with a fluorescent chemical substance. The clearing method in accordance with an aspect of the present invention enables, when using a high-resolution microscope or a super-resolution microscope for example, obtaining super-resolution images in areas at a depth up to about 50 µm to 200 µm. This depth is restricted by the working distance of an objective lens at present. Therefore, observation of deeper areas would become available in the future. Since the clearing method in accordance with an aspect of the present invention does not cause swelling of biological materials, the above depth is actually deeper than those of previous methods (which cause swelling of biological materials) which enable imaging at about the same depth. Therefore, the clearing method in accordance with an aspect of the present invention makes it possible to image the localization of fluorescence in deeper areas.

The biological material may be a material fixed with paraformaldehyde or the like for microscopy or may be an unfixed material, but is preferably a fixed material. The fixing may be performed by, for example, a known method.

The thickness of the biological material is not particularly limited, and may be, for example, 1 µm to 10 mm, preferably 100 µm to 2 mm. Since the clearing agent and the mounting agent for use in an aspect of the present invention enable imaging in deeper areas, the present invention is applicable even when the thickness is 0.1 mm or more.

### (Advantages)

The following are main advantages of embodiments of the present invention.

1) As will be described later in Examples, fine structures of biological materials are not likely to collapse. 2) Transparency is higher than when using a previous clearing agent, because biological materials do not shrink. This makes it possible to observe various fluorescent proteins and fluorescent substances in tissues in super-deep areas. In addition, the fine structures of the biological materials are unlikely to change. 3) The biological materials are unlikely to be broken because they do not swell. Therefore, an aspect of the present invention is applicable to fragile biological materials (e.g., brains of newborn mice). 4) Even when the biological materials are placed in an isotonic solution such as PBS again, both strength and cellular morphology thereof are not affected. Due to such high reversibility, imaging utilizing clearing and tissue staining may be used in combination. 5) Biological materials can be cleared in a much shorter time than previous clearing methods (e.g., CLARITY and CUBIC). 6) Since swelling of biological materials is not caused, the areas that can be imaged are actually deeper than those achieved by previous clearing agents, provided that the working distance of an objective lens of a microscope is restricted. 7) High-resolution imaging is achieved while preserving fluorescence of various fluorescent proteins and fluorescent chemical substances. 8) Good transparency is achieved without having to perform complicated steps such as acrylamide gel crosslinking and lipid removal. 9) Fluorescence of fluorescent proteins and fluorescent chemical substances can be more accurately observed because no autofluorescence signals are produced. 10) The mounting agent of an aspect of the present invention is bright and allows the best use of high-resolution or super-resolution microscopes especially in deep areas as compared to previous water-soluble mounting agents (e.g., ProLong Gold and RIMS) and therefore greatly contributes to development of imaging in deep areas.

### (Other applications)

The clearing using a clearing agent of an aspect of the present invention is reversible. Therefore, a cleared biological material can recover its uncleared state by, for example, immersing the biological material in an equilibrium salt solution and thereby removing components of the clearing agent. Examples of the equilibrium salt solution include: an equilibrium salt solution buffered with phosphate such as PBS and HBSS; an equilibrium salt solution buffered with Tris hydrochloride salt (TBS): basal media for cell culture such as MEM, DMEM, and Ham's F-12; and the like.

Furthermore, the mounting agent of an aspect of the present invention causes little damage to biological materials. Therefore, a mounted biological material can recover its unmounted state by, for example, immersing the biological material in an equilibrium salt solution and thereby removing components of the mounting agent. Examples of the equilibrium salt solution include those described earlier.

The clearing agent for use in an aspect of the present invention does not cause denaturation etc. of proteins and the like in the biological material when the biological material is cleared or when the cleared biological material recovers its uncleared state. Therefore, the antigenicity of the proteins and the like in the biological material is also kept unchanged. In addition, the mounting agent of an aspect of the present invention causes little damage to the biological material. Therefore, it is possible to employ the following arrangement, for example: a cleared biological material is observed under a microscope, and thereafter the biological material recovers its uncleared state before being subjected to detailed analyses using a publicly known tissue staining or immunostaining method.

### [2. Method for observing biological material]

A method for observing a biological material in accordance with an aspect of the present invention includes a step of observing, under a microscope, a biological material in a solution (mounting agent) that contains a non-ionic organoiodine compound and that has a refractive index higher than 1.48 (such a step is hereinafter referred to as "observing step B"). In one embodiment of the method for observing a biological material in accordance with the present invention, the observing step B is performed on a biological material that has not been subjected to the clearing step. In another embodiment, the observing step B is performed after the pre-treatment step, which was described earlier in the [1. Method for clearing biological material] section. In a still another embodiment, the observing step B is performed on a biological material that has been cleared by a method other than the clearing method of an aspect of the present invention. Alternatively, in a yet another embodiment, the biological material may be a biological material that has been cleared by the clearing method of an aspect of the present invention.

As described earlier, the observing step B of an aspect of the present invention reduces spherical aberrations and thus enables imaging in deeper areas. Therefore, for example, in a case of a biological material which requires no clearing, the biological material may be subjected to the observing step B of an aspect of the present invention without being cleared. This enables imaging deep inside the biological material. The biological material which requires no clearing is, for example, a relatively thin biological material. Examples include: cells (e.g., cultured cells); oocytes (e.g., oocytes of mammals such as mice); brains of invertebrates (e.g., Drosophila); animal and plant tissues sliced to a thickness of about 50 µm or less; and yeast and E. coli suspensions; and the like.

Even in a case of a biological material that has been cleared by a method other than the clearing method of an aspect of the present invention, the biological material may be subjected to the observing step B of an aspect of the present invention and thereby spherical aberrations may be reduced. This may enable imaging deep inside the biological material. The method other than the clearing method of an aspect of the present invention is, for example, the method disclosed in any of Non-Patent Literatures 1 to 4 and Patent Literature 1. There is no particular limitation on a biological material to be subjected to the method, provided that the biological material can be cleared by the method other than the clearing method of an aspect of the present invention.

The mounting agent has already been described in the [1. Method for clearing biological material] section. Specifically, to the non-ionic organoiodine compound, solvent, buffer, additive, refractive index, and the like of the mounting agent, the descriptions of the mounting agent for use in the method for clearing a biological material are applicable.

The observing step B is performed in a similar manner to the (Observing step A) of the [1. Method for clearing biological material] section.

The biological material to be subjected to the observing step B may be optionally subjected to a visualizing step, such as staining or marking, prior to the observing step. The visualizing step has already been described in the (Visualizing step) of the [1. Method for clearing biological material] section. Furthermore, the biological material may be subjected to any of the steps described in the (Other steps) in the [1. Method for clearing biological material] section.

### [3. Clearing agent]

The clearing agent in accordance with an aspect of the present invention is a clearing agent for biological materials and is a solution that contains a detergent and a non-ionic organoiodine compound. Examples of the clearing agent in accordance with an aspect of the present invention include the earlier-described clearing agent A and clearing agent B. These clearing agents have already been described in the [1. Method for clearing biological material] section.

The clearing agent in accordance with an aspect of the present invention may be suitably used in the earlier-described "method for clearing a biological material".

### [4. Mounting agent]

The mounting agent in accordance with an aspect of the present invention is a mounting agent for use in observing a biological material under a microscope and is a solution that contains a non-ionic organoiodine compound and that has a refractive index higher than 1.48. Examples of the mounting agent in accordance with an aspect of the present invention include the earlier-described mounting agents. The mounting agent has already been described in the [1. Method for clearing biological material] section.

The mounting agent in accordance with an aspect of the present invention may be suitably used in the earlier-described "method for clearing a biological material" and "method for observing a biological material".

### [5. Kit for observation of biological material]

A kit for observation of a biological material in accordance with an aspect of the present invention is a kit to be used to clear a biological material and observe the cleared biological material under a microscope, and includes: a first solution (clearing agent A) that contains a detergent and a non-ionic organoiodine compound; and a second solution (mounting agent) that contains a non-ionic organoiodine compound and that has a refractive index higher than 1.48. The kit for observation of a biological material in accordance with an aspect of the present invention may preferably further include at least one solution (clearing agent B) that contains a detergent and a lower concentration of a non-ionic organoiodine compound than the clearing agent A. The clearing agent A, the mounting agent, and the clearing agent B have already been described in the [1. Method for clearing biological material] section.

The kit for observation of a biological material in accordance with an aspect of the present invention preferably further includes a "manual for the kit". The "manual for the kit" provides, for example, instructions of how to perform a step of clearing a biological material using the clearing agent A, more preferably additionally using the clearing agent B, and a step of observing the biological material using the mounting agent, as described in the [1. Method for clearing biological material] section.

The kit for observation of a biological material in accordance with an aspect of the present invention may further include some tool, reagent, and/or the like necessary for clearing and observation of the biological material. For example, the kit may include at least one selected from: treatment containers for use in the earlier-described clearing step and forceps for biological materials (e.g., tweezers); pre-treatment solutions which are described earlier; glass slides; glass coverslips; immersion liquids; and an equilibrium salt solution for the biological material to recover its uncleared state after the observation.

The kit for observation of a biological material in accordance with an aspect of the present invention may be suitably used in the earlier-described "method for observing a biological material".

### [6. Kit for clearing biological materials]

A kit for clearing a biological material in accordance with an aspect of the present invention is a kit to be used to clear a biological material, and includes a solution (clearing agent A) that contains a detergent and a non-ionic organoiodine compound. The kit for clearing a biological material in accordance with an aspect of the present invention may preferably further include at least one solution (clearing agent B) that contains a detergent and a lower concentration of a non-ionic organoiodine compound than the clearing agent A. The clearing agent A and the clearing agent B have already been described in the [1. Method for clearing biological material] section.

The kit for clearing a biological material in accordance with an aspect of the present invention preferably further includes a "manual for the kit". The "manual for the kit" provides, for example, instructions of how to perform a method of clearing a biological material using the clearing agent A, more preferably additionally using the clearing agent B, as described in the [1. Method for clearing biological material] section.

The kit for clearing a biological material in accordance with an aspect of the present invention may further include some tool, reagent, and/or the like necessary for clearing of the biological material. For example, the kit may include at least one selected from: treatment containers for use in the earlier-described clearing step and forceps for biological materials (e.g., tweezers); pre-treatment solutions which are described earlier; and an equilibrium salt solution for the cleared biological material to recover its uncleared state.

The kit for clearing a biological material in accordance with an aspect of the present invention may be suitably used in the earlier-described "method for clearing a biological material".

### [7. Recap]

The clearing method in accordance with an aspect of the present invention for clearing a biological material includes a clearing step A of clearing the biological material by immersing the biological material in a solution that contains a detergent and a non-ionic organoiodine compound.

The clearing method in accordance with an aspect of the present invention may further include an observing step of observing the biological material, which has been cleared, under a microscope in a state in which the biological material is immersed in a solution that contains a non-ionic organoiodine compound and that has a refractive index higher than 1.48.

The clearing method in accordance with an aspect of the present invention may further include a clearing step B of clearing the biological material before the clearing step A by immersing the biological material in at least one solution that contains a detergent and a non-ionic organoiodine compound whose concentration is lower than that of the solution used in the clearing step A. Here, in a case where two or more solutions are used each of which contains a detergent and a non-ionic organoiodine compound whose concentration is lower than that of the solution used in the clearing step A, the biological material is sequentially immersed in the two or more solutions in ascending order of concentration of the non-ionic organoiodine compound.

The clearing method in accordance with an aspect of the present invention may further include an immersing step of immersing the biological material in a solution that contains a detergent and no non-ionic organoiodine compound before the biological material is cleared.

An observing method in accordance with an aspect of the present invention for observing a biological material includes an observing step of observing the biological material under a microscope in a state in which the biological material is immersed in a solution that contains a non-ionic organoiodine compound and that has a refractive index higher than 1.48.

The observing method in accordance with an aspect of the present invention may further include an immersing step of immersing, before the observing step, the biological material in a solution that contains a detergent and no non-ionic organoiodine compound.

The clearing agent in accordance with an aspect of the present invention is a clearing agent for clearing a biological material and is a solution containing a detergent and a non-ionic organoiodine compound.

The mounting agent in accordance with an aspect of the present invention is a mounting agent for use in observing a biological material under a microscope and is a solution that contains a non-ionic organoiodine compound and that has a refractive index higher than 1.48.

The kit in accordance with an aspect of the present invention for observing a biological material is a kit for use in clearing a biological material and observing the biological material under a microscope and includes: a first solution that contains a detergent and a non-ionic organoiodine compound; and a second solution that contains a non-ionic organoiodine compound and that has a refractive index higher than 1.48.

The following will provide Examples to more specifically describe embodiments of the present invention. As a matter of course, the present invention is not limited to Examples provided below, but details of the present invention can be realized in various manners. Further, the present invention is not limited to the embodiments described above, and it may be varied in various ways within the scope of the appended claims. Thus, an embodiment based on a proper combination of technical means disclosed in different embodiments is encompassed in the technical scope of the present invention. In addition, the present specification encompasses the contents of the specification and/or drawings of Japanese Patent Application No. 2015-055325, to which the present application claims priority. Furthermore, all of the publications and patents cited in the present specification are incorporated herein by reference in their entirety.

### Examples

### [Procedures]

### (Reagents)

SeeDB2G was prepared using Omnipaque 350 (e.g., Daiichi-Sankyo; 75.5% (w/v) or 56.2% (w/w) iohexol in Tris-EDTA buffer). SeeDB2S was prepared by dissolving iohexol powder (available as Histodenz (Sigma-Aldrich) or Nycoden (Axis-shield)) at 70.4% (w/w) in a mixture of 10 mM Tris-Cl (pH7.6) and 0.267 mM EDTA. Note that SeeDB2S was prepared based on "w/w", not "w/v". Solutions 1 and 2 were prepared by diluting Omnipaque 350 in H₂O and adding saponin (Nacalai-tesque or Sigma-Aldrich) at 2% (w/v). Saponin shows different levels of browning across lots. Therefore, the inventors of the present invention used lots with a less brownish color because the brown pigment reduces light transmittance. Reagents for CLARITY were purchased from Wako Pure Chemical Industries, Ltd. Refractive indices were determined using an Abbe refractometer (Erma Inc.) and white light source. SeeDB2G and SeeDB2S are collectively referred to as SeeDB2.

### (Optical clearing of mouse brain tissues using SeeDB2)

PFA-fixed mouse brain samples were cleared using 5 mL Eppendorf tube with gentle rotation (not more than 4 rpm) using an overhead rotater (TAITEC) at room temperature (25°C). First, fixed tissue samples were incubated in 2% saponin (Sigma) in PBS overnight. Samples were next incubated in 1:2 mixture of Omnipaque 350 (Daiichi-Sankyo) and H₂O with 2% saponin (Solution 1) for 6 to 10 hours, and then in 1:1 mixture of Omnipaque 350 and H₂O with 2% saponin (Solution 2) for 6 to 10 hours. Finally, samples were incubated in Omnipaque 350 with 2% saponin (Solution 3) for 12 hours or longer. Thin brain slices could be cleared with shorter incubation schedules. Some samples (e.g., lipid-rich tissue and large samples) may be further cleared by prolonged incubation in Solution 3. For clearing with SeeDB2S, samples were further incubated in SeeDB2S with 2% saponin (Solution 4) for 12 hours or longer. Cleared tissues were transferred to SeeDB2G or SeeDB2S without saponin for imaging. For long-term storage of samples, 0.01% sodium azide may be added as a preservative. The addition of chemical preservatives (e.g., DABCO) is not recommended for fluorescent protein samples, because they often quench fluorescence. Samples can be stored at room temperature under protection from light. The cleared sample should not be exposed to the air for long time, because the surface may become sticky due to water evaporation.

### (SeeDB)

SeeDB (80.2% (wt/wt) fructose, and 0.5% (vol/vol) α-thioglycerol in H₂O) was prepared as previously reported (Reference Literatures: Non-Patent Literature 4 and Ke, MT., and Imai, T (2014) Curr Protoc Neurosci 66, Unit 2 22). In confocal and two-photon microscopy using an upright microscope, index-matched 2,2'-thiodiethanol solution in H₂O was used for immersion (Reference Literature: Non-Patent Literature 4).

### (CUBIC)

ScaleCUBIC-1 (25% (w/w) urea, 25% (w/w) N,N,N',N'-tetrakis(2-hydroxypropyl)ethylenediamine, and 15% (w/w) Triton X-100 in H₂O) and ScaleCUBIC-2 (50% (w/w) sucrose, 25% (w/w) urea, 10% (w/w) 2,2',2"-nitrilotriethanol, and 0.1% (v/v) Triton X-100 in H₂O) were prepared as reported previously (Reference Literature: Non-Patent Literature 2).

### (CLARITY)

The inventors used the passive clearing protocol of CLARITY (Reference Literature: Tomer et al. (2014). Nat Protoc 9, 1682-1697), because this can reportedly better preserve morphology than the original protocol based on electrophoretic lipid removal (Reference Literature: Non-Patent Literature 3). Anesthetized mice were intracardially perfused with 20 ml ice-cold PBS and then with 20 ml HM solution (4% acrylamide, 0.05% bisacrylamide, 4% PFA, and 0.25% VA-044 in PBS). Excised brain was then transferred to 20 ml HM solution and incubated at 4°C for 1 to 2 days. Samples were then incubated at 37°C for polymerization overnight. Then, brain was excised from the hydrogel and washed in SBC buffer (4% SDS in 200 mM boric acid buffer, pH 8.5) for 1 day. To make brain slices, samples were embedded in 4% low-melting agarose and sliced using a vibratome microslicer (Dosaka EM). For clearing, samples were incubated in SBC buffer with gentle shaking. Clarified samples were washed in 0.1% Triton-X100 in PBS and mounted in FocusClear (CelExplorer Labs). Reagents for CLARITY were purchased from Wako Pure Chemical Industries, Ltd.

### (ScaleS)

ScaleS0 (20% sorbitol, 5% glycerol, 1 mM methyl-β-cyclodextrin, 1 mM γ-cyclodextrin, 1% N-acetyl-Lhydroxyproline, and 3% DMSO), ScaleS1 (20% sorbitol, 10% glycerol, 4M urea, and 0.2% Triton X-100), ScaleS2 (27% sorbitol, 2.7M urea, 0.1% Triton X-100, and 8.3% DMSO), ScaleS3 (36.4% sorbitol, 2.7M urea, and 0.1% DMSO), and ScaleS4 (40% sorbitol, 10% glycerol, 4M urea, 0.2% Triton X-100, and 20% DMSO) were prepared as reported previously (Reference Literature: Hama, H., et al. (2015). Nature neuroscience 18, 1518-1529).

### (Mouse strains)

All animal experiments were approved by the Institutional Animal Care and Use Committee of the RIKEN Kobe Institute. Wild-type (ICR, C57BL/6N, and BDF1), *Thy1-YFP* line G (JAX #014130), *Thy1-YFP* line H (JAX #003782) (Reference Literature: Feng, G. et al. (2000). Neuron 28, 41-51), I7*-IRES-ECFP* under *MOR23* promoter (RIKEN BRC #RBRC02931) (Reference Literature: Imai et al. (2006). Science 314, 657-661), MOR29-AIRES-*ECFP*/*MOR29B-EYFP* BAC transgenic line (Reference Literature: Tsuboi, A., et al. (2011). The European journal of neuroscience 33, 205-213), and *R26-H2B-EGFP* (Acc .No.CDB0238K; http://www.cdb.riken.jp/arg/reporter_mice.html) (Reference Literature: Abe et al. (2011). Genesis 49, 579-590) mice in C57BL/6N background were used for imaging experiments. Floxed *Grin1* mice (Reference Literature: Tsien, J.Z., et al. (1996). Cell 87, 1317-1326) backcrossed to C57BL/6J (JAX #005246) were bred to ICR mice for *in utero* electroporation. Both males and females were used for imaging experiments.

### (Mouse brain samples)

To obtain tissue samples, mice were deeply anesthetized with an overdose i.p. injection of Nembutal (Dainippon Sumitomo Pharma), followed by intracardiac perfusion with 4% paraformaldehyde (PFA) in phosphate buffered saline (PBS). Excised brains or embryos were fixed with 4% paraformaldehyde (PFA) in PBS at 4°C overnight. Brain slices (0.2 to 2 mm thick) were cut using a microslicer (Dosaka EM).

### (In utero electroporation)

Homozygous floxed Grin1 mice and wild-type mice in C57BL/6J × ICR mixed background were used to perform single-cell knockout analyses of Grin1 coding for NR1, an essential subunit for NMDA receptor function. pCAG-loxP-Neo-loxP-tdTomato (1 µg/µL), pCAG-loxP-Neo-loxP-EYFPGephyrin (0.5 µg/µL), and pCAG-Cre (0.5 µg/µL) were co-electroporated to the L5 pyramidal neurons at E12 and analyzed at P22. In utero electroporation was performed as reported previously using forceps-type electrodes (5 mm diameter) and a CUY21EX electroporator (BEX) (Reference Literature: Ke, M.T., Fujimoto, S. & Imai, T. (2013). Nat Neurosci 16, 1154-1161). The backbone plasmidvector, pCAG-CreERT2 and pCAFNF-DsRed were gifts from C. Cepko (Addgene plasmid #14797 and #13771).

### (Quantification of transparency)

Quantification of transparency was determined by measuring light transmittance of postnatal day 21-24 cerebral cortex. Because white matter scatters unevenly, the inventors removed thalamus and hippocampus from the hemi-forebrain samples and only used gray matter (cerebral cortex). Light transmittance of clearing agents and cleared brain tissues was determined using a spectrophotometer (Hitachi, U-5100). Transmittance along the medial-lateral axis of the hemi-brain cortical sample was quantified.

### (Measurements of sample size changes)

The sample size changes during optical clearing were quantified as reported previously (Reference Literature: Non-Patent Literature 4) using adult mouse hemibrain samples. Flat surface of the brain was placed on dishes and images were taken from top under a stereo microscope. Linear expansion was determined based on square root of area size changes.

### (Quantification of morphological damages)

To quantify morphological changes during optical clearing, the inventors used lateral dendrites of mitral cells (which were aspiny and smooth in vivo). To sparsely label mitral cells, AlexaFluor 488- or 647-dextran (10 kDa) were electroporated into the mouse olfactory bulb *in vivo* as reported previously (Reference Literature: Ke, M.T., Fujimoto, S. & Imai, T. (2013). Nat Neurosci 16, 1154-1161). *In vivo* images (AlexaFluor 488) were taken with two-photon microscopy in anesthetized mice. After clearing and fluorescence imaging (AlexaFluor 647) of olfactory bulb, lateral dendrites of mitral cells were segmented at perisomatic site, branch point, end point, and the end of the image field. Then the minimal distance between the two ends of the segments (D) and the length of the traced dendrites (d) were determined using Neurolucida software (MBF Bioscience). The tortuosity index was defined as d/D, as has been previously reported (Reference Literature: Ke, M.T., Fujimoto, S. & Imai, T. (2013). Nat Neurosci 16, 1154-1161).

### (Quantification of fluorescence in brain tissue sections)

To quantitate the stability of fluorescent proteins in tissue, *Thy1-YFP-G* or *Thy1-YFP-H* mouse brain samples were used. Twenty pm-thick sections were prepared using a cryostat (Leica, CM3050S) and collected on glass slides. Sections were fixed with 4% PFA in PBS for 15 min and washed in PBS. Sections were then incubated in optical clearing agents for up to 24 hours. Fluorescence intensities of fluorescent proteins in tissues were quantified using an inverted fluorescence microscope (Leica, DMI6000B) with a cooled CCD camera.

### (Spectral analyses of recombinant fluorescent proteins and chemical fluorescent dyes)

E.coli JM109 (DE3) strain was transfected with ₚRSET_{A}-ECFP, EGFP, EYFP, or tdTomato vector. The obtained bacterial cultures were incubated in 2×YT medium with ampicillin for 16 hours at 30°C and then for 20 hours at 25°C. Bacterial pellets were treated with four freeze and thaw cycles in the presence of lysozyme (Sigma- Aldrich) and benzonase (Novagen) and recombinant proteins were purified using Ni-NTA Spin Kit (QIAGEN). Purified recombinant proteins were diluted in various clearing agents and incubated overnight at room temperature. Excitation and emission spectrum were quantified using a fluorescence spectrophotometer, model F2700 (Hitachi). For the quantification of Alexa dyes, Alexa dye-conjugated donkey anti-mouse antibodies (Life Technologies) were used. Excitation and emission wavelength used to determine emission and excitation curves, respectively, were as follows (in nm): ECFP (435, 480), EGFP (480, 515), EYFP (505, 535), tdTomato (545, 590), Alexa 488 (480, 530), Alexa 555 (540, 580), Alexa 647 (640, 675).

### (Immunostaining of mouse brain slices)

Immunostaining and counterstaining may be performed prior to SeeDB2 clearing. To facilitate antibody penetration, the samples were incubated in 2% saponin in PBS with gentle rotation (not more than 4 rpm) for 24 hours at 4°C. Saponin better preserves morphology than Triton X-100, a commonly used detergent for immunostaining. The samples were then transferred to 3 ml blocking buffer (0.5% (w/v) skim milk, 0.25% (w/v) fish gelatin, 2% (w/v) saponin, 0.5% (w/v) Triton X-100 (optional), and 0.05% (w/v) sodium azide in PBS) in 5 ml Eppendorf tube. Blocking was performed with gentle rotation for 24 hours at 4°C. Reaction with primary antibodies (in 3ml blocking buffer using 5ml Eppendorf tube) was performed for 24 hours on a rotator at 4°C. Mouse anti-GAD67 (Millipore, MAB5406), mouse anti-Reelin (Millipore, MAB5364), mouse anti-Bassoon (Abcam, AB82958), and rabbit anti-Homerl (Synaptic Systems, 160003) were used at 1/200 dilution. After three washes (2 hours each) in washing buffer (2% saponin, 0.5% Triton X-100 in PBS), samples were incubated with secondary antibodies in 3 ml of blocking buffer (antibodies were at 1/200 dilution) for 12 to 16 hours on a rotator at 4°C. Donkey anti-mouse Alexa647 (Life Technologies) and anti-rabbit Alexa555 (Life Technologies) were used at 1/200 dilution. DAPI (Life Technologies; 1/500 dilution) or NeuroTrace 640/660 Deep-Red Fluorescent Nissl Stain (Lif Technologies; 1/200 dilution) was applied during the secondary antibody reaction when necessary. The samples were then washed three times (2 hours each) in washing buffer. The antibody-stained brain samples were then cleared and mounted in SeeDB2 as described above.

### (HEK293T cell samples for SR-SIM)

HEK293T cells kept in FluoroBrite DMEM with 10% FBS were seeded in 35 mm poly-D-lysine coated glass bottomed dishes (MatTek) and then transfected with 0.4 pg pCAG-CreER^{T2} (addgene plasmid #14797, a gift from C. Cepko) and 3.6 µg pcDNA5/FRT-loxP-3×SV40 polyA-loxP-gapEGFP. The gapEGFP contains N-terminal 20 amino acid sequence from gap43, required for palmitoylation, and localize to the plasma membrane (membrane EGFP). CreER was used for leaky recombination without tamoxifen. Twenty-four hour after transfection, cells were loaded with 500 nM MitoTracker Red CMXRos for 45 min. Cells were then fixed with 4% PFA for 15 min and then stained with DAPI in 0.2% Triton X-100 in PBS for 15 min. Cells were then incubated in Solution 1 for 30 min, Solution 2 for 30 min, Solution 3 for 30 min, Solution 4 for 30min, and SeeDB2S for 30 min or longer before SR-SIM imaging.

### (HEK293T cell samples for PALM and dSTORM)

For PALM imaging, mEos2-Lifeact-7 (addgene plasmid #54809, a gift from M. Davidson) plasmid was transfected to HEK293T cells in 35 mm glass bottomed dish (MatTek). Twenty-four hours after transfection, cells were fixed with pre-warmed 4% PFA in PBS for 15 min and then cleared with SeeDB2S as described above. For dSTORM samples, HEK293T cells were fixed with pre-warmed 4% PFA in PBS, immunostained with anti-α-Tubulin (DM1A, Sigma; 1:500), and then with AlexaFluor 647 Donkey Anti-Mouse IgG (Thermo Fisher, A31571).

### (Confocal imaging using inverted microscopes)

Imaging setup was reported previously (Reference Literatures: Non-Patent Literature 4 and Ke, M.T., and Imai, T. (2014). Curr Protoc Neurosci 66, Unit 2 22). The cleared whole-mount brain samples were dipped in a glass-bottomed dish filled with SeeDB2. To mount brain slices, silicone rubber sheet with appropriate thickness (Togawa rubber) and 0.170 ±0.005 mm thick glass coverslip (Marienfeld, No. 1.5H) was used. Confocal images were taken using an inverted microscopes, FV1000 (Olympus), TCS SP8X (Leica Microsystems), LSM780 (Carl Zeiss), or LSM880 (Carl Zeiss). In FV1000, the inventors used laser diode (405 nm), Multi-Ar (457, 488, and 515 nm), and He-Ne (543 and 633 nm) laser lines. A 20× (Olympus, UPLSAPO20X, NA 0.75, WD 0.60 mm) air objective lenses were used. Because optical axial scale is shortened in SeeDB2G (RI 1.46), the actual depth was determined by multiplying an apparent depth by 1.46. The calibrated depths are shown in all figures. In high-resolution imaging, a 100× (Olympus, UPLSAPO 100XO, NA 1.40, WD 0.13 mm) oil-immersion objective lens was used. A commercial immersion oil, Immersol 518F (Carl Zeiss) or Immersion oil type F (Olympus) was used for the imaging (RI 1.518). In TCS SP8X, the inventors used white Light Laser for excitation, and the inventors used HyD detectors for detecting fluorescence signals. A 40× oil-immersion (Leica, HC PL APO 40x OIL CS2, NA 1.3, WD 0.24 mm) and a 63× glycerol-immersion (Leica, HC PL APO 63x/1.30 Glyc CORR CS2, NA 1.3, WD 0.30 mm) objective lenses were used. Type G (RI 1.46) and Type F (RI 1.518) immersion media were used for SeeDB2G and SeeDB2S samples, respectively. In LSM780/880, images were acquired using a 20× dry lens (Plan-Apochromat 20x/0.8 M27, NA 0.80, WD 0.55) or a 40× water-immersion lens (P-Apochromat 63x Oil DIC, NA 1.40, WD 0.19) and GaAsP detectors.

### (Light-sheet microscopy)

Light-sheet microscopy of cleared mouse brain was performed using a commercialized light-sheet microscope, model TCS SP8 DLS based on inverted confocal microscopy (Leica Microsystems). Adult *Thy1- YFP-H* mouse brain slices (2 mm thick) was cleared with SeeDB2G and then cut with a razor blade into smaller pieces for sample setup. Two pieces of glass coverslips were vertically stood with glue on a glass-bottomed dish and the dissected brain sample was held in between. Samples were immersed in SeeDB2G for imaging. Light-sheet illumination was at 514 nm (Ar laser) and EYFP signals were detected with an objective lens, a TwinFlect mirror devise (HC APO L10x/0.30 W DLS) and sCMOS camera. Because the commercialized objective lens is designed for water-immersion (RI 1.33), focal plane is different when used in SeeDB2G with RI 1.46. Therefore, the inventors used a customized mirror devise (Leica Microsystems) to detect images at focal plane in SeeDB2G solution.

### (Confocal and two-photon imaging using an upright microscope)

Imaging chambers for an upright microscope were prepared as reported previously (Reference Literature: Ke, M.T., and Imai, T. (2014). Curr Protoc Neurosci 66, Unit 2 22). One to six mm thick silicone rubber sheet (Togawa rubber) was used to make an imaging chamber. Samples and SeeDB2 were placed in the chamber, and they were sealed with a glass-bottomed Petri dish (Reference Literature: Ke, M.T., and Imai, T. (2014). Curr Protoc Neurosci 66, Unit 2 22). For SeeDB2G samples, immersion medium (67% (v/v) 2,2'-thiodiethanol in H₂O, RI 1.46) was added to the glass-bottomed dish. An upright confocal/two-photon microscope (Olympus, FV1000MPE) with a motorized stage (Sigma-Koki) was used for the fluorescence imaging. A custom-made 25× objective lens optimized for high-index samples (Olympus, NA 0.9, WD 8.0 mm, designed for refractive index 1.41-1.51) were used to image SeeDB2 samples (a similar lens is now commercialized from Olympus as XLSLPLN25XGMP). LD473 laser was used for one-photon excitation of EYFP. InSight DeepSee Dual (Spectra-Physics) was used for two-photon excitation of EGFP (920 nm) and EYFP (950 nm).

### (Super-resolution fluorescence microscopy)

STED microscopy was performed using a commercial STED microscope, model TCS SP8 STED 3X (Leica Microsystems) with an oil-immersion objective lens (Leica, HC PL APO 100x/1.40 OIL STED WHITE, NA 1.40, WD 0.13 mm). EYFP was excited with Multi Ar laser (514 nm) and 592 nm STED laser was used for 2D STED. Fluorescence was detected with a HyD detector with a gated STED function. SP8-HyVolution was performed using TCS SP8X (Leica Microsystems) with a 63x oil-immersion (Leica, HC PL APO 63x OIL CS2, NA 1.4, WD 0.14 mm). ECFP and EYFP were excited with laser diode (442 nm) and White Light Laser, respectively. Fluorescence signals were detected using HyD detectors and processed with Huygens (Scientific Volume Imaging B. V.). SR-SIM was performed using commercialized setup, model ELYRA PS.1 (Carl Zeiss) with a 100× oil-immersion objective lens (Carl Zeiss, alpha Plan-Apochromat 100× / 1.46 oil, NA 1.46, WD 0.11 mm). Grating was performed using 3 rotations. The averaging number was 2. In FV1000 with FV-OSR (Olympus), a 100× oilimmersion objective lens (Olympus, UPLSAPO 100XO, NA 1.40, WD 0.13 mm) was used. Fluorescence was detected with GaAsP detectors and averaging was performed 25 to 30 times. In SD-OSR (Olympus) based on CSU-W1 (Yokogawa), a 100× oil-immersion objective lens (Olympus, UPLSAPO 100XO, NA 1.40, WD 0.13 mm) was used. EYFP was excited with 488 nm laser. In LSM880 with Airyscan (Carl Zeiss), oil-immersion objective lenses, 63× oil (NA 1.4, WD 0.19 mm) and 100× oil (NA 1.46, WD 0.11 mm) were used. Fluorescence was detected with an Airyscan detector and super-resolution mode (pinhole size, 1.25 AU). EGFP, EYFP, and Alexa Fluor 488 were excited at 488 nm, and tdTomato was excited at 561 nm. PALM/dSTORM imaging were performed with ELYRA PS.1 (Carl Zeiss) using a 100× oil-immersion objective lens (alpha Plan-Apochromat 100× Oil DIC M27, NA 1.46, WD 0.11 mm) and TIRF, HILO, or Epi illumination mode. Due to high RI, TIRF illumination mode does not allow for TIRF imaging with SeeDB2S, and illumination light penetrated into samples. In the PALM imaging, mEos2 was illuminated with 405 nm and 561 nm lasers.

### (PSF analyses)

PSF was determined as reported previously (Reference Literature: Non-Patent Literature 4) using 0.1 µm (for confocal and Airyscan) or 0.04 µm FluoSpheres yellow-green fluorescent microspheres (Thermo Fisher, F8803 and F8795). Fluorescent beads were embedded in 1% (for 100 nm beads) or 4% (for 40 nm beads) agarose, and then permeated with clearing agents. Full width at half maximum (FWHM) was determined by Gaussian fitting using Fiji plugin MetroloJ. Sample size was 20 each in all PSF analyses.

### (Sea response analyses)

To evaluate fluorescence intensity using axial confocal scan, rhodamine 6G (Tokyo Chemical Industry) was dissolved in clearing agents. The dye solutions were sealed with a glass coverslip (Marienfeld, No. 1.5H) in the imaging chamber and imaged with an inverted confocal microscope, FV1000 (Olympus), using a 100× (Olympus, UPLSAPO 100XO, NA 1.40, WD 0.13 mm) oil-immersion lens.

### (Image processing and quantification)

Volume rendering of fluorescence images were performed using Neurolucida (MBF Bioscience) and IMARIS (bitplane). Huygens deconvolution software for Leica TCS SP8X was used for STED and SP8-HyVolution images. VAST Lite (https://software.rc.fas.harvard.edu/lichtman/vast/) originally developed for serial EM images was used for reconstruction of super-resolution images (Reference Literature: Kasthuri, N., et al. (2015). Cell 162, 648-661). Traced image stacks were exported to Matlab (Mathworks) to make surface meshes and then visualized with ParaView (http://www.paraview.org/). Neurolucida and Neurolucida 360 with AutoNeuron and AutoSpine functions (MBF Bioscience) were used for semi-automatic neuronal tracing and quantification of dendritic spines in mouse pyramidal neurons. Average axon diameter was determined with Neurolucida for 50 pm-long axon segments. These procedures are summarized in Fig. 13.

### (Statistical analyses)

Prism5 software (GraphPad) and R (http://www.r-project.org/) were used for statistical analysis. In C of Fig. 2, two-tailed Welch's t-test was used assuming normal distribution. In E of Fig. 1, the inventors used non-parametric tests based on non-normal distribution. Data are shown as mean ± SEM for technical replicates. Mean ± SD was used to indicate biological variations. Sample sizes (specified in figure legends) were determined based on the distribution of the data in pilot experiments. Quantification of neuronal morphology was non-blinded; however, to avoid any potential biases among sample types, all the labelled neurons were quantified in E of Fig. 1. In Fig. 12, brightly-labelled neurons were chosen for the analysis, oblique dendrites extending from the proximal part of apical dendrites were traced, and full-length of the dendrites was quantified.

### [Results]

### (A quick and efficient optical clearing using a non-ionic organoiodine compound)

To obtain high-resolution images deep inside tissues, it is necessary to minimize both light scattering and spherical aberrations (A of Fig. 6). Previous efforts by the group of the inventors and others have indicated that optimal refractive index of the clearing agents is 1.45 to 1.55 (Reference Literature: Non-Patent Literature 4). To minimize spherical aberrations, the RIs of samples and immersion media should be the same. Because the minimal distance between theoretically resolvable points (d) increases as numerical aperture (NA) increases (d = 0.61λ/NA; according to the Rayleigh criterion), and achievable NA increases as refractive index of immersion media (n) increases (NA = n sinα; α is for the half opening angle of the objective), objective lenses for high-resolution imaging are typically designed for oil-immersion (RI 1.518). Immersion oil is also designed to match the RI of glass coverslips (RI = not higher than 1.52). Thus, the ideal optical clearing agent for high-resolution imaging should have RI 1.518. Previously, 97% TDE of an RI of 1.515 has been used as a mounting medium for this purpose; however, utility of TDE has been limited, because it quenches most fluorescent proteins and some of commonly-used chemical dyes (Reference Literature: Non-Patent Literature 6). Previous clearing agents, such as SeeDB (RI 1.49), could not reach this RI due to limited solubility of fructose (Reference Literature: Non-Patent Literature 4). Commonly-used mounting media with glycerol (RI 1.47) also cannot achieve this RI. Commercialized high-RI mounting media cause shrinkage and/or have quenching issues. Therefore, large-scale volumetric super-resolution imaging has been a long-standing challenge. During a screen for high-index water-soluble agents, the inventors focused on iodide-containing chemicals. The inventors found, for example, that 70.4% (w/w) iohexol in water reach RI 1.518 (C of Fig. 6). Therefore, the inventors tried to develop a novel clearing agent and mounting agent using a non-ionic organoiodine compound such as iohexol.

More recently, iohexol solution (RI 1.46) with Tween-20 and phosphate buffer was used to mount CLARITY samples as an affordable substitute to FocusClear (named RIMS) (Reference Literature: Non-Patent Literature 5). However, neither iohexol nor RIMS alone could efficiently clear thick brain samples by simple immersing, and instead caused shrinkage of samples (A to C of Fig. 1). This was likely because the iohexol solution did not efficiently penetrate into tissues. Therefore, the inventors tested its clearing performance in combination with various detergents, including dodecyl sodium sulfate (SDS), Tween (Registered Trademark) 20, Nonidet (Trademark) P-40, Triton X-100 (Trademark), and saponin at 0.5 to 2%, and found that 2% saponin, known as a weak non-ionic detergent, can most efficiently facilitate clearing by iohexol without introducing morphological damages (F of Fig. 6). Tween 20, Nonidet P-40, and saponin reduced sample deformation and/or quenching/loss of fluorescent proteins, as compared to SDS and Triton X-100. Lower concentrations of Triton X-100 could be used for relatively thin samples. Saponin and Triton X-100 more efficiently facilitated tissue clearing by iohexol. The inventors also tested various buffers to maintain transparency of brain samples, because brain samples immersed in non-buffered iohexol solution gradually returned opaque by unknown reasons. As a result, the inventors found that low concentrations of Tris-EDTA buffer can more efficiently maintain excellent transparency of samples when combined with iohexol (G of Fig. 6). When water or PBS was used instead of Tris-EDTA buffer, excellent transparency was not obtained and the transparency decreased as time passed. In an optimized clearing protocol, tissue samples were serially incubated in lower to higher concentrations of iohexol in 2% saponin and Tris buffer, and finally equilibrated in 70.4% (w/w) iohexol solution in Tris-EDTA buffer (RI 1.518), named SeeDB2S (S for Super-resolution) (C of Fig. 1). For glycerol-immersion objective lenses, the inventors also formulated a protocol to equilibrate samples at RI 1.46 (56.2% (w/w) or 75.5% (w/v) iohexol solution; named SeeDB2G, G for glycerol-immersion lens). Due to lower viscosity and sufficient transparency, SeeDB2G was useful for large tissue samples, though SeeDB2S is more useful for high resolution imaging using oil-immersion lenses. Transmission curves of cerebral cortex samples demonstrate that SeeDB2S and SeeDB2G clear samples better than SeeDB, which is a known clearing agent, and RIMS, which is a mounting media (B of Fig. 1). Even nasal bone was well cleared with SeeDB2S (D of Fig. 1). Total incubation time required was a few hours (for relatively thin samples) to 2 days (adult half brain samples), which was much quicker than other clearing protocols, such as CLARITY and CUBIC (C of Fig. 1). Like SeeDB, SeeDB2 is non-hazardous and did not introduce any fragility to tissues.

### (SeeDB2 maintains fine neuronal morphology)

To obtain genuine high-resolution images, it is crucial to maintain fine cellular structures of samples during clearing process. This is not always well considered in other clearing protocols (Reference Literature: Non-Patent Literature 4). For example, ScaleA2 causes sample swelling, whereas BABB causes shrinkage, and both caused deformation of neuronal processes (Reference Literature: Non-Patent Literature 4). Even recently-developed clearing methods, CLARITY, CUBIC, and ScaleS, are accompanied by transient sample swelling, although the final sizes of these samples were comparable to the original sample (Reference Literatures: Non-Patent Literatures 2 to 5; Hama, H., et al. (2015). Nature neuroscience 18, 1518-1529; Tomer, R., et al. (2014). Nat Protoc 9, 1682-1697). In contrast, in SeeDB2G/S protocols of an aspect of the present invention, sample sizes were maintained during the entire clearing process (C of Fig. 1). As a result, sample integrity was well preserved in various samples.

To evaluate fine morphological changes during clearing, the inventors quantified the tortuosity of aspiny mitral cell dendrites sparsely labelled in the mouse olfactory bulb (Reference Literature: Non-Patent Literature 4). After clearing with CLARITY, CUBIC, or ScaleS, dendrite tortuosity increased compared to *in vivo,* showing deformation, even though the sample size after clearing was restored to the original size (E of Fig. 1). This is no surprise, given that high-order protein structures, such as cytoskeletons, do not swell or shrink isotopically. In contrast, samples cleared with SeeDB2 did not show any evidence of morphological deformation (E of Fig. 1). Thus, SeeDB2 allows for the accurate and quantitative analysis of fine neuronal morphology of samples of, for example, neurons.

### (SeeDB2 preserves more fluorescence with lower autofluorescence signals)

In high-resolution fluorescence imaging, it is necessary to obtain photons from smaller volumes per pixel than in low resolution imaging, indicating that fluorophores have to be bright and stable. The inventors therefore examined the stability of various fluorophores in SeeDB2 and existing mounting agents. Four fluorescent proteins, ECFP, EGFP, EYFP, and tdTomato were found to be stable and showed similar excitation and emission spectra in SeeDB2 compared to those in PBS (A of Fig 2). Furthermore, fluorescence signals were quite stable during long-term storage (1 month or longer) in SeeDB2 without accumulation of autofluorescence signals (B to E of Fig. 7). In contrast, these fluorescent proteins were dramatically quenched in 97% 2,2'-thiodiethanol (TDE), currently the best mounting agents for super-resolution microscopy in terms of RI (Reference Literature: Non-Patent Literature 6). A commercial mounting agent, ProLong Gold also reduced fluorescence of EYFP substantially. Three chemical fluorophores, Alexa Fluor 488, 555, and 647 were also found to be stable in SeeDB2S, although 97% TDE reduced fluorescence of Alexa Fluor 488 considerably (A of Fig. 7). The inventors obtained similar results with mouse brain sections expressing EYFP (B of Fig. 2). Thus, both fluorescent proteins and chemical fluorescent dyes are stable in SeeDB2S, compared to commonly used mounting agents for confocal and high-resolution microscopy.

The inventors previously noted that fructose-based clearing agent, SeeDB, gradually accumulated autofluorescence in tissues, because it reacts with endogenous amines (known as Maillard reaction) (Reference Literature: Non-Patent Literature 4). In contrast, SeeDB2 did not produce autofluorescence signals (B of Fig. 7). Furthermore, fluorescence signals were quite stable during long-term storage in iohexol solution (C to E of Fig. 7). *Thy1-YFP* line G (*Thy1-YFP-G*) mouse brain samples maintained sufficient levels of EYFP signals even after 1 month or longer storage at room temperature in SeeDB2G (C of Fig. 7). Recombinant fluorescent proteins were also stable in SeeDB2S at least for one month at room temperature (E of Fig. 7).

These points are advantageous to image faint fluorescence signals in large tissues. In the cerebral cortex of *Thy1-YFP-H* animals, fine dendritic tufts extending from layer V pyramidal neurons were better visualized in SeeDB2 than in SeeDB, CUBIC, and CLARITY, due to high fluorescence levels and low autofluorescence signals (H of Fig. 1, B of Fig. 7, D of Fig. 7). Light-sheet microscopy enabled fast and large-scale imaging of the cerebral cortex (A of Fig. 2). Due to low autofluorescence signals, SeeDB2G was also powerful to image all nuclei of other types of samples (such as a whole embryo (E9.5) carrying the *R26-H2B-EGFP* knock-in allele, in which H2B-EGFP is ubiquitously expressed at relatively low level) (B and C of Fig. 2) (Reference Literature: Abe et al. (2011). Genesis 49, 579-590). Other types of organs were also well cleared for fluorescence imaging (C of Fig. 2). As saponin facilitates penetration of macromolecules, SeeDB2 was also compatible with nuclear counterstains and antibody staining of tissue slices. DAPI was penetrated into 1.5 mm thick adult brain slices (D of Fig. 2); Antibodies could stain up to 200 µm depth in adult mouse brain slices (E of Fig. 2), which is sufficient for high-resolution mapping of small-scale circuitry.

### (Resolution and brightness are maintained throughout depths in SeeDB2S)

To evaluate performance of SeeDB2S in high-resolution fluorescence imaging, the inventors first used a confocal microscope with oil-immersion lens (NA 1.40, WD 0.13 mm) in order to quantify spatial resolution by point-spread-function (PSF) analysis of fluorescent microspheres (100 nm diameter) embedded in agarose gels. Previously, thick brain tissues have been imaged using two-photon microscopy. However, because resolution (d) increases as excitation wavelength (λ) increase (d = 0.61λ/NA), one-photon excitation provides twice better resolution than in two-photon microscopy under the same NA (A of Fig. 8). Under the confocal microscopy, an oil-immersion objective lens provides better resolution than a water-immersion lens, because NA increases as RI of immersion medium increase (NA = n sinα) (A of Fig. 8). Thus, optical clearing and one-photon imaging are advantageous than in two-photon microscopy in terms of resolution.

Next, the inventors examined resolution and brightness in various clearing agents and mounting agents using an oil-immersion objective lens. As expected, PSF analyses demonstrated that high resolution was maintained throughout depths when mounted in the refractive index-optimized SeeDB2S (A and B of Fig. 3). However, in other mounting agents with lower RI, resolution was worse, due to spherical aberrations. Z axial resolution was more severely affected by the RI mismatch. Improvement of Z-axial resolution in SeeDB2S is important in circuit tracing, as it becomes easier to dissect crossing-over of multiple fibers along z-axis. To evaluate depth-dependent changes in fluorescence brightness, the inventors performed confocal axial scans of Rhodamine 6G dye solutions from the interface of the glass coverslip (Sea response) (Reference Literature: Hell, S. et al. (1993). Journal of Microscopy 169, 391-405). With SeeDB2S, the Rhodamine fluorescence level was maintained throughout all depths. However, in non-optimized mounting agents, the brightness gradually reduced as depth increased, due to spherical aberrations (C of Fig. 3). It is noted here that SeeDB2G has a refractive index of 1.46 and SeeDB2S has a refractive index of 1.518. The use of SeeDB2S, which has a refractive index matching those of glass coverslip and/or immersion oil, provided high resolution and high brightness.

### (High-resolution confocal imaging of densely-labelled neuronal circuitry)

Using conventional confocal microscopy and high-NA oil immersion objective lenses, the inventors could easily obtain high-resolution images of SeeDB2S-treated mouse brain slices up to the working distance (WD) of objective lenses (D and E of Fig. 3). Because resolution was constant throughout depth in SeeDB2S, fine dendritic spines were clearly visualized throughout the depth (up to 200 µm). For thinner samples, using the combination of SeeDB2G and high-NA glycerol-immersion lens with longer WD (NA 1.30, WD 0.30 mm), the inventors could obtain high-resolution images of olfactory sensory neuron axons converging to a glomerulus in the olfactory bulb at a single axon resolution (F of Fig. 3).

This resolution was sufficient to trace the densely-labelled neuronal circuitry. The inventors obtained high-resolution fluorescence images from a 238 µm × 127 µm × 132 µm block of cerebral cortex from *Thy1-YFP-H* mouse brain slice samples cleared with SeeDB2S (G of Fig. 3). Using semi-automated neuronal tracing software, the inventors could extract and reconstruct in 3D a near complete dendritic wiring diagram of hippocampal CA1 pyramidal neurons (H of Fig. 3, B of Fig. 8). Thus, using SeeDB2S, it is possible to easily perform high-resolution neuronal mapping that was previously performed by array tomography (AT), a time-consuming approach (Reference Literature: Micheva, K.D., and Smith, S.J. (2007). Neuron 55, 25-36).

### (Super-resolution microscopy of mouse brain slices)

To resolve the geometry of dendritic spines and synapses beyond the diffraction barrier, one of the super-resolution microscopy, stimulated emission depletion (STED) microscopy has been often used. However, due to spherical aberrations, standard STED microscopy can only obtain super-resolution images on the surface of specimens. To obtain super-resolution images deep inside tissues, it is necessary to use special optical correction (Reference Literature: Urban, N.T. et al. (2011). Biophys J 101, 1277-1284) or two-photon excitation STED (Reference Literature: Ding, J.B. et al. (2009). Neuron 63, 429-437).

Here the inventors used commercialized STED microscopy to image *Thy1-YFP-H* mouse brain slices cleared with SeeDB2S. The inventors could obtain super-resolution images up to 100 µm in depth, the upper limit set by the working distance (WD) of an objective lens (A of Fig. 4). Indeed, spine neck diameters were often below the theoretical diffraction limit (up to 200 nm), as has been reported previously (Reference Literature: Tonnesen, J. et al. (2014). Nature Neuroscience 17, 678-+). The inventors could visualize fine geometry of dendritic spines and spine necks, an essential information to understand synaptic functions. For a sample embedded in a common commercial mounting agent, ProLong Gold, the inventors could only visualize the surface area of the sample, due to spherical aberrations. Furthermore, because fluorescence signals were lower in this sample, the inventors had to use a stronger laser power for this sample. As a result, samples were often photobleached before obtaining z-stack images of super-resolution fluorescence images. Thus, SeeDB2S dramatically expand the imaging depth possible for STED microscopy.

The inventors also tested commercialized super-resolution systems based on deconvolution of Airy disc images (FV-OSR and Airyscan). As a result, again, the inventors could obtain super-resolution images of dendritic spines up to the depth allowed by the WD of an oil-immersion objective lens (not greater than 100 µm or not greater than 170 µm, B and C of Fig. 4, A and B of Fig. 9). Due to minimal photobleaching, Airyscan system was useful to obtain super-resolution images at a large scale in 3D (C of Fig. 4).

### (Three-dimensional super-resolution microscopy in cell biology)

Super-resolution microscopy has become a popular approach in cell biology. However, even in cell biology applications, spherical aberration is a big problem limiting the imaging depth to the very surface. For example, a commercial super-resolution structured illumination microscope (SR-SIM) can resolve the sub-diffraction images up to just a few micrometers, which is not sufficient to visualize an entire thickness of even a single cell (10 to 20 µm). HEK293T cells labelled with DAPI, membrane EGFP, and MitoTracker could be fully resolved in PBS. The same sample mounted in ProLong Gold or TDE was photobleached during imaging, because fluorescence levels were much lower in these media, and required higher laser power. However, when mounted in SeeDB2S of an aspect of the present invention, the inventors could obtain super-resolution images of these structures throughout thickness of these cells (E of Fig. 5). Fine filopodia extending from the plasma membrane were resolved throughout the entire depth.

### (Deep-tissue super-resolution microscopy using SeeDB2S)

The inventors next evaluated the performance of SeeDB2S in super-resolution microscopy. The inventors tested a commercialized stimulated emission depletion (STED) microscope with an oil-immersion objective lens (NA 1.40, WD 0.13 mm). To determine the lateral PSF at various depths, the inventors used 40 nm diameter fluorescence microspheres embedded in agarose gel. In SeeDB2S and 97% TDE, 50-60 nm FWHM in x-y was maintained up to 100 µm depth; however, in ProLong Gold and PBS, resolution was quickly degraded and fluorescent microspheres were no longer visible at 20 µm or greater depth (A of Fig. 5).

The inventors next imaged *Thy1-YFP-H* mouse brain slices cleared with SeeDB2S under the same STED laser conditions. The inventors could obtain sub-diffraction images up to 110 µm in depth, the upper limit set by the WD of an objective lens (B of Fig. 11). The inventors could visualize the fine geometry of dendritic spine heads and spine necks, which are essential information to understand synaptic functions (C of Fig. 5). In addition, the inventors occasionally observed filopodia extending from existing spine heads, which is difficult to resolve using conventional microscopy (C of Fig. 5). To evaluate resolutions at various depths in brain tissues, the inventors determined spine neck diameters, which are known to be thinner than the diffraction limit (Reference Literature: Tonnesen, J., et al. (2014). Nature Neuroscience 17, 678-+). The inventors found no difference in spine neck diameters quantified at superficial (not greater than 30 µm) and deep areas of brain slices (not greater than 110 µm) (D of Fig. 5). Thus, SeeDB2S enables STED microscopy at a greater depth than previously possible under standard mounting conditions. It should be noted that deep-tissue STED microscopy has only been achieved by two-photon excitation STED (Reference Literature: Ding, J.B., et al. (2009). Neuron 63, 429-437; Moneron, G., and Hell, S.W. (2009). Opt Express 17, 14567-14573) or by manual optical calibration (Reference Literature: Urban, N.T., et al. (2011). Biophys J 101, 1277-1284); for the latter, it is unrealistic to manually correct the refractive index mismatch at each z position in order to obtain large-scale 3D images.

### (Volumetric super-resolution imaging for cell biology)

A different type of super-resolution microscopy, structured illumination microscopy (SIM) allows multicolor imaging and has often been used for cell biology applications. Here the inventors used a commercial super-resolution (SR) SIM system with an oil-immersion lens (NA 1.46, WD 0.11 mm) to image SeeDB2S samples. HEK293T cells (10 µm or thinner) labelled with membrane EGFP, MitoTracker, and DAPI could be fully resolved with 3D SR-SIM in SeeDB2S, but not in PBS. For example, fine filopodia extending from the plasma membrane were better resolved in SeeDB2S (E of Fig. 5).

### (Volumetric super-resolution imaging for connectomics)

Large-scale super-resolution imaging may be particularly powerful for connectomics applications combined with fluorescent proteins. Here the inventors used a new type of commercial super-resolution microscopy, Airyscan, which is based on multi-array GaAsP detectors and pixel reassignment (Reference Literature: Huff, J. (2015). Nat Methods 12). Due to the relatively high photon budget and low photo bleaching among various super-resolution microscopies, Airyscan was useful for largescale imaging, although the resolution was not as good as STED. In PSF analysis, the high resolution (not greater than 150 nm in xy; not greater than 360 nm in z) was maintained throughout depth using an oilimmersion lens (NA1.40, WD 0.19 mm) (A of Fig. 11).

The inventors compared images of the same region of a *Thy1-YFP-H* mouse brain slice, using two photon microscopy, confocal microscopy, and Airyscan. Because the longer excitation wavelength is the lower resolution become (d = 0.61λ/NA), two photon microscopy is not good for high-resolution imaging. Airyscan best resolved dense neuronal circuits labelled with EYFP (B of Fig. 11). Filopodia and thin spines, often missed in two-photon microscopy, were reliably identified with Airyscan. The "stubby" spines often described in two-photon imaging studies were not observed in Airyscan (at least in L5 neurons) and are most likely projection artifacts of thin/mushroom spines due to low axial resolution (Reference Literature: Tonnesen, J., et al. (2014). Nature Neuroscience 17, 678-+). The inventors could also obtain large-scale super-resolution images with SD-OSR (Reference Literature: Hayashi, S., and Okada, Y. (2015). Mol Biol Cell 26, 1743-1751) and SP8-HyVolution (A to C of Fig. 10).

Due to excellent axial resolution, dendritic spines extending along z-axis could be reliably detected with SeeDB2S, but not with other mounting media, due to spherical aberrations (C of Fig. 11). SeeDB2S was also useful for the tracing and quantification of thin axons. In the corpus callosum, L2/3 callosal axons labelled by in utero electroporation were well resolved in SeeDB2S, but not in ScaleS (D of Fig. 11). Improvement of axial resolution in SeeDB2S is particularly important in circuit tracing, as it becomes easier to dissect crossing-over of multiple fibers along x-axis. In the axon bundles of olfactory sensory neurons, unmyelinated thin axonal fibers (200 to 300 nm in diameter, consistent with earlier EM studies) labelled with MOR29B-IRES-EYFP transgene were well resolved and dissected in SeeDB2 (E of Fig. 11 and C of Fig. 10). In the large-scale super-resolution images of the cerebral cortex (100 µm thick, adult *Thy1-YFP-H* mouse), the inventors could reliably identify dendritic spines and axonal boutons at all depth (C of Fig. 4) and reconstruct them in 3D (D of Fig. 4). Thus, the combination of SeeDB2S and super-resolution microscopy is useful for large-scale synapse mapping.

### (Comprehensive synapse mapping in dendrites of NMDA receptor-deficient neurons)

Many cognitive and mental disorders are caused by synaptic abnormalities, and thus the super-resolution mapping of synapses can become a powerful tool for understanding of learning-related synaptic changes and its pathology. The inventors mapped excitatory and inhibitory synapses in wild-type and NMDA receptor-deficient L5 pyramidal neurons (in which an essential NR1 subunit was knocked-out) using *in utero* electroporation (A and B of Fig. 12) (Reference Literature: Tsien, J.Z., et al. (1996). Cell 87, 1317-1326). Excitatory synapses are known to localize at the tip of mature dendritic spines in L5 neurons and its size is correlated with the spine head diameter. Based on spine head diameter, the inventors classified dendritic spines into three types: filopodia (less than 250 nm), thin spine (250 to 500 nm), and mushroom spine (greater than 500 nm) (C of Fig. 12). To visualize postsynaptic structures of inhibitory synapses, the inventors used EYFP-gephyrin (Reference Literature: Chen, J.L., et al. (2012). Neuron 74, 361-373). EYFP-gephyrin puncta were found in dendritic shaft and a subset of spine heads, and these two types were unambiguously distinguished in super-resolution images of an aspect of the present invention (A and B of Fig. 12). The inventors focused their analysis on 100-200 µm-long unbranched oblique dendrites extending from the trunk of apical dendrites, from the proximal to distal end in full, to avoid any biases in quantification.

In the NR1-deficient neurons, total spine density and average spine length were unchanged (D and E of Fig. 12), but the distribution of spine head diameters was different from wild type (C, D, and F of Fig. 12). Increase in average spine head diameter is consistent with an earlier study using cortex-specific NR1 knockout mice (Reference Literature: Ultanir, S.K., et al. (2007). Proceedings of the National Academy of Sciences of the United States of America 104, 19553-19558). As for EYFP gephyrin puncta, density in the shaft was not affected significantly; however, density in spine heads was increased in KO (G of Fig. 12). The inventors also found that EYFP-gephyrin puncta tended to accumulate at large spine heads in the NR1 KO neurons (H and I of Fig. 12). Thus, the NMDA receptor knockout affects not only excitatory synapses, but also inhibitory synapses directly or indirectly. Recruitment of inhibitory synapses to large spines in the NR1-deficient neurons may be due to an activity-dependent homeostatic action of inhibitory synaptogenesis in these spines (Reference Literature: Baho, E. & Di Cristo, G. (2012). Journal of Neuroscience 32, 911-918).

### Industrial Applicability

The present invention can be used in a case where, for example, a biological material is cleared and then observed under a microscope.

## Claims

1. A method for clearing a biological material, said method comprising:
a clearing step A of clearing the biological material by immersing the biological material in a solution that contains a detergent and a non-ionic organoiodine compound.

2. The method as set forth in claim 1, further comprising:
an observing step of observing the biological material, which has been cleared, under a microscope in a state in which the biological material is immersed in a solution that contains a non-ionic organoiodine compound and that has a refractive index higher than 1.48.

3. The method as set forth in claim 1 or 2, further comprising:
a clearing step B of clearing the biological material before the clearing step A by immersing the biological material in at least one solution that contains a detergent and a non-ionic organoiodine compound whose concentration is lower than that of the solution used in the clearing step A,
in a case where two or more solutions are used each of which contains a detergent and a non-ionic organoiodine compound whose concentration is lower than that of the solution used in the clearing step A, the biological material being sequentially immersed in the two or more solutions in ascending order of concentration of the non-ionic organoiodine compound.

4. The method as set forth in any one of claims 1 through 3, further comprising:
an immersing step of immersing the biological material in a solution that contains a detergent and no non-ionic organoiodine compound before the biological material is cleared.

5. A method for observing a biological material, said method comprising:
an observing step of observing the biological material under a microscope in a state in which the biological material is immersed in a solution that contains a non-ionic organoiodine compound and that has a refractive index higher than 1.48.

6. The method as set forth in claim 5, further comprising:
an immersing step of immersing, before the observing step, the biological material in a solution that contains a detergent and no non-ionic organoiodine compound.

7. A clearing agent for clearing a biological material, said clearing agent being a solution containing a detergent and a non-ionic organoiodine compound.

8. A mounting agent for use in observing a biological material under a microscope, said mounting agent being a solution that contains a non-ionic organoiodine compound and that has a refractive index higher than 1.48.

9. A kit for use in clearing a biological material and observing the biological material under a microscope, said kit comprising:
a first solution that contains a detergent and a non-ionic organoiodine compound; and
a second solution that contains a non-ionic organoiodine compound and that has a refractive index higher than 1.48.
